# EUROPÄISCHE PATENTSCHRIFT

(11) **EP 2 307 359 B1**
(45) Veröffentlichungstag und Bekanntmachung des Hinweises auf die Patenterteilung: **04.01.2012**
(21) Anmeldenummer: 09780778.8
(22) Anmeldetag: 17.07.2009
(51) Int. Cl.: C07C 279/00

(54) **BLENDS ENTHALTEND EPOXIDHARZE UND MISCHUNGEN VON AMINEN MIT GUANIDIN-DERIVATEN**
BLENDS CONTAINING EPOXY RESINS AND MIXTURES OF AMINES WITH GUANIDINE DERIVATIVES
MÉLANGES CONTENANT DES RÉSINES ÉPOXY AINSI QUE DES MÉLANGES D'AMINES ET DE DÉRIVÉS DE GUANIDINE

(30) Priorität: 22.07.2008 EP 08160857; 08.07.2009 WO PCT/EP2009/058699
(43) Veröffentlichungstag der Anmeldung: 13.04.2011
(73) Patentinhaber: BASF SE, 67056 Ludwigshafen (DE)
(72) Erfinder: DAUN, Gregor, 69151 Neckargemünd (DE); WITTENBECHER, Lars, 40545 Düsseldorf (DE); HENNINGSEN, Michael, 67227 Frankenthal (DE); FLICK, Dieter, 67459 Böhl-Iggelheim (DE); GEISLER, Joerg-Peter, 55441 Bingen (DE); SCHILLGALIES, Juergen, 47445 Moers (DE); JACOBI, Erhard, 65510 Hünstetten (DE)
(86) Internationale Anmeldenummer: PCT/EP2009/059238
(87) Internationale Veröffentlichungsnummer: WO 2010/010048

(56) Entgegenhaltungen:
- EP-A- 1 358 997
- WO-A-02/42349
- WO-A-02/070623
- WO-A-03/002667
- WO-A-03/045801
- WO-A-2004/020506
- DATABASE WPI Week 200526 Thomson Scientific, London, GB; AN 2005-246390 XP002543486 & JP 2005 067209 A (HIRAOKA SHOKUSEN KK) 17. März 2005 (2005-03-17)
- DATABASE WPI Week 200321 Thomson Scientific, London, GB; AN 2003-213909 XP002543487 & JP 2002 240205 A (HIRAOKA SHOKUSEN KK) 28. August 2002 (2002-08-28)

## Beschreibung

Der Gegenstand der vorliegenden Erfindung betrifft ein Blend enthaltend ein oder mehrere Epoxidharze und eine Mischung, die 0,3 bis 0,9 Aminäquivalent, pro Äquivalent Epoxid des eingesetzten Epoxidharzes, an einer Härterkomponente a) und als Härterkomponente b) eine Verbindung der Formel I enthält, ein Verfahren zur Herstellung dieses Blends, die Verwendung des erfindungsgemäßen Blends zur Herstellung gehärteten Epoxidharzes sowie ein mit dem erfindungsgemäßen Blend gehärtetes Epoxidharz.

Die Aminhärtung von Epoxidharzen wird in unterschiedlichsten Bereichen genutzt. So wird die Aminhärtung der Epoxidharze bei Klebstoffen, zur Aushärtung von Gießharzen in speziellen Formen als auch zur Versiegelung von Oberflächen und vor Umwelteinflüssen zu schützenden Bauteilen eingesetzt.
Ein spezielles großes Anwendungsgebiet der Aminhärtung von Epoxidharzen ist die Herstellung von faserverstärkten Kunststoffen. Faserverstärkte Kunststoffe werden eingesetzt als Werkstoffe für Kraftfahrzeuge, Flugzeuge, Schiffe und Boote, für Sportartikel und Rotorblätter von Windkraftanlagen.
Die Herstellung großer Bauteile stellt besondere Anforderungen an den Härter bzw. die Härtermischung, da während der Verarbeitungszeit die Viskosität nicht so stark ansteigen darf, dass entweder die Fasern nicht ausreichend benetzt werden oder aber die Form nicht vollständig gefüllt ist, bevor das Epoxidharz nicht mehr verarbeitbar ist. Gleichzeitig soll die Zykluszeit (Verarbeitung und Härtung) nicht negativ beeinflusst werden. Es besteht daher ein großer Bedarf an Mischungen, die in der Lage sind, die Aushärtung des Epoxidharzes in jeglichen Systemen exakt zu kontrollieren und einzustellen.

H. Klein beschreibt in "Huntsman Amine Overview", Huntsman, June 19, 2007, Beijing Epoxy Conference, dass primäre und sekundäre Diamine und Polyetheramine zur Härtung von Epoxidharzen allgemein eingesetzt und verwendet werden können. Ein Blend in dem die Härterkomponente a) im Bereich von 0,3 bis 0,9 Aminäquivalent, pro Äquivalent Epoxid des eingesetzten Epoxidharzes, eingesetzt und in der die Härterkomponenten b) eine Verbindung der Formel I ist, wird jedoch nicht beschrieben.

B. Burton, D. Alexander, H. Klein, A. Garibay Vasquez und C. Henkee beschreiben in der Produktbroschüre "Epoxy formulations using Jeffamine Polyetheramines", Huntsman, April 21, 2005 die stöchiometrische Verwendung von Polyetheraminen bzw. Mischung aus Polyetheraminen und anderen Diaminen wie Isophorondiamin (IPDA) als eine besondere Form der Aminhärtung von Epoxidharzen. Hierbei handelt es sich um Zweikomponentensysteme, bei denen das Amin bzw. die Aminmischung unmittelbar vor der Härtung dem Epoxidharz zugesetzt wird und zwar in Mengen, die genauso viele aktive Aminfunktionen in der Aminmischung wie aktive Epoxid-Funktionen in den Epoxiden enthalten.
In Härterformulierungen aus Polyetheraminen und IPDA bewirkt letzteres einerseits eine höhere Geschwindigkeit der Härtung und anderseits werden in den ausgehärteten Harzen höhere Glasübergangstemperaturen beobachtet, was zu einer höheren Temperaturbeständigkeit der ausgehärteten Produkte führt die bei einigen Anwendungen wie beispielsweise der Herstellung von Rotorblättern gefordert werden, als das ein einer Härtung bei vergleichbarer Temperatur mit reinem Polyetheramin der Fall ist.

Verglichen mit der Härtung von Epoxidharzen durch Polyetheramine bedingt der Zusatz von IPDA neben einer höheren Glasübergangstemperatur der ausgehärteten Harze jedoch auch eine raschere Aushärtung, was mit einem schnelleren Anstieg der Viskosität einhergeht. Dadurch wird die Zeit, in der das Blend aus Epoxidharz und Härter/Härtermischung noch verarbeitbar ist, verringert. Nachteilig bei solchen Härtermischungssystemen ist daher, dass die Herstellung großer Bauteile, wie Rotorblätter, möglicherweise nicht gelingt, da der Infusionsprozess aufgrund der Viskositätsentwicklung unvollständig bleibt.

Die Geschwindigkeit der stöchiometrischen Aushärtung von Epoxidharzen mit Aminen kann auch gesteigert werden, indem dem Blend tertiäre Amine zugesetzt werden, die als Beschleuniger fungieren. Auch dieser Zusatz führt meist zu einem schnelleren Anstieg der Viskosität bei Raumtemperatur und zu niedrigeren Topfzeiten. Unter dem Begriff Topfzeit oder auch Gelierzeit ist eine Kenngröße zu verstehen, die üblicherweise benutzt wird, um die Reaktivität verschiedener Harz/Härter- und/oder Harz/Härtermischungs-Kombinationen zu vergleichen. Die Messung der Topfzeit/Gelierzeit (To) wird nach der Vorschrift von ASTM D 2471-99 beschrieben und ist eine Methode zur Charakterisierung der Reaktivität von Laminiersystemen mittels einer Temperaturmessung. Je nach Anwendung haben sich Abweichungen von den dort beschriebenen Parametern (Menge, Prüfbedingungen und Messmethode) etabliert, die zu seiner Topfzeit A (ToA) und einer Topfzeit B (ToB) führen.

Dabei wird die Topfzeit A (ToA) wie folgt bestimmt:
100 g des Blends, enthalten Epoxidharz und Härter bzw. Härtgemisch werden in einem Behälter (üblicherweise ein Pappbecher) gefüllt. In diesen Blend wird ein Temperaturfühler eingetaucht, der in bestimmten Zeitabständen die Temperatur misst und abspeichert. Sobald dieses Blend erstarrt ist, wird die Messung beendet und die Zeit bis zum erreichen der Maximaltemperatur wird ermittelt. Für den Fall, dass die Reaktivität eines Blend zu gering ist, wird diese Messung bei erhöhter Temperatur durchgeführt. Neben der Topfzeit muss auch immer die Prüftemperatur angegeben werden.

Die Topfzeit B (ToB) wird wie folgt bestimmt:
5g des Blends enthaltend Epoxidharz und Härter /Härtergemisch werden in einem 5mL Penicillinglas bei einer gegebenen Prüftemperatur (nicht adiabatisch) gefüllt. Im Blend bewegt sich ein runder Probenstempel (∅ 11,8 mm) auf und ab (1 mm/sec). Wenn ein entsprechender Widerstand (ca. 5 kPa) erreicht wird schaltet die Stoppuhr ab.

Als Beispiele solcher oben beschriebenen Beschleuniger werden Triethanolamin, Benzyldimethylamin, 2,4,6-Tris(dimethylaminomethyl)phenol und Tetramethylguanidin in US-A 4,948,700 Spalte 10 genannt. Die prinzipielle Eignung von tetra- und penta-Alkylguanidinen als Härter von Epoxidharzmischungen ist in US 3,308,094 beschrieben. Der Einsatz von Tetramethylguanidin als tertiäres Amin mit sehr geringer katalytischer Aktivität wird auch in US-A 6,743,375 in Spalte 19 erwähnt. US-A 6,743,375 lehrt den Fachmann jedoch, dass Tetramethylguanidin ein vergleichsweise langsamer Beschleuniger ist. Ein Blend in dem die Härterkomponente a) im Bereich von 0,3 bis 0,9 Aminäquivalent, pro Äquivalent Epoxid des eingesetzten Epoxidharzes, eingesetzt wird und die Härterkomponente b) eine Verbindung der Formel I ist, wird jedoch nicht beschrieben.

Die Härtung von Epoxiden mit Aminen wird unter anderem bei Infusionstechnologien eingesetzt. Hierbei werden Di- und Polyepoxidharze mit Aminen und Polyetheraminen unmittelbar vor dem Einfüllvorgang zum Blend gemischt, das Blend bei Temperaturen von 20°C - 50°C in die jeweilige Form eingesaugt und danach bei Formtemperaturen von 55°C - 90°C zur Reaktion gebracht, wodurch es zur Aushärtung des Blends kommt. Die Geschwindigkeit des gesamten Prozesses ist dabei abhängig von der Dauer des Einfüllschrittes, der eigentlichen Infusion, und von der Dauer der Aushärtung. Der Einfüllvorgang kann umso schneller erfolgen, je geringer die Viskosität des Blends ist. Die Verringerung der Viskosität eines vorgegebenen Blends kann durch die Erhöhung der Temperatur während des Einfüllvorgangs erfolgen, wodurch dieser prinzipiell schneller wird. Die Erhöhung der Temperatur während des Einfüllvorgangs zur Verringerung der Viskosität ist allerdings nur bei wenig reaktiven Aminen, wie z.B. Polyetheraminen sinnvoll. Der Nachteil der alleinigen Verwendung wenig reaktiver Amine, wie z.B. Polyetheramine, ist die langsame Reaktion dieser Komponente mit dem Epoxidharz, wodurch der Aushärtungsvorgang langsam erfolgt. Die Dauer der Aushärtung kann verkürzt werden durch die Verwendung besonders reaktiver Amine wie beispielsweise IPDA. Bei Anwesenheit dieser reaktiven Amine muss die Infusion allerdings bei tiefen Temperaturen erfolgen, da die Viskosität einer Mischung aus Polyetheramin und IPDA bei Temperaturen > 40°C derart schnell ansteigt, dass eine vollständige Tränkung der Fasermatten nicht mehr gewährleistet werden kann.

Bei der Verwendung von Infusionstechnologien wie der "Vacuum assisted resin transfer molding"- Technologie (VARTM) für die Produktion großer Bauteile, kann eine lange Topfzeit des Blends enthaltend Epoxidharze und Amine im Bereich mehrerer Stunden bei Raumtemperatur erforderlich sein, um einen problemlosen Einfüllvorgang zu gewährleisten. Diese lange Topfzeit kann durch die Verwendung wenig reaktiver Polyetheramine erreicht werden, wie sie in WO-A 2004/020506, Seite 14-17 beschrieben werden. Ein ausschließlicher Einsatz von aktiven Härtern wie IPDA ist aus dem Stand der Technik für die Infusionstechnologie bei großen Bauteilen nicht bekannt. Der Nachteil des Einsatzes ausschließlich wenig reaktiver Polyetheramine in der Infusionstechnologie liegt in den extrem langen Aushärtzeiten bei erhöhter Temperatur, die eine Steigerung der Produktivität verhindern und gleichzeitig erhöhten Energieeinsatz erfordern.

Eine Verbesserung des Infusionsprozesses mit Blends enthaltend Epoxidharze und Amine erfolgt dann, wenn die Viskosität des Blends während des Einfüllvorgangs niedrig ist, bzw. wenn der Einfüllvorgang bedingt durch eine längere Topfzeit des verbesserten Blends bei höheren Temperaturen und damit bei einer niedrigeren Viskosität erfolgen kann, als es bei den bisher bekannten Blends aus Epoxidharzen, Polyetheraminen und IPDA der Fall ist.
Die Aufgabe eines verbesserten Verfahrens zur Herstellung solcher Formkörper wäre es, bei Temperaturen von beispielsweise 60°C oder mehr eine vergleichbare oder höhere Aushärtungsgeschwindigkeit gegenüber dem Stand der Technik zu zeigen. Solche Verfahren wären gerade für die Fertigung großer Bauteile sehr geeignet, da bei vergleichbarer oder kürzerer Aushärtungsgeschwindigkeit, die Verarbeitungszeit bei Raumtemperatur verlängert oder die Verarbeitung bei höheren Temperaturen möglich wäre, ohne dass es zu einer vorzeitigen Aushärtung des Blends käme und somit eine vollständige und gleichmäßige Aushärtung möglich wäre.

GB-A 2005-246390 sowie JP-A 2005/067209 beschreiben eine Polyolefinharzfolie, die neben Polyolefinschichten auch eine Schicht aus Textilfaser enthält, wobei die Polyolefinschichten aus unterschiedlichen Polyolefinmischungen und nicht-flammbaren Partikeln bestehen. Die nicht-flammbaren Partikel werden mit Hilfe von aushärtbaren Harzen auf die Polyolefinschichten aufgeklebt. In keinem der beiden Literaturstellen werden weder Härterkomponenten noch eine Mischung aus Aminen mit einer Funktionalität ≥ 2 und einer zweiten Härterkomponente, die unter die Formel I fällt genannt noch deren Mischungesverhältnisse offenbart. GB-A 2005-246390 sowie
JP-A 2005/067209 beschreiben des Weiteren nicht, dass ein Blend, das die Härterkomponenten a) und b) enthält und in den beschriebenen Mengenverhältnissen zueinander vorliegt, so steuerbar ist, dass die Aushärtegeschwindigkeit (Topfzeit) des Blends gegenüber den Härtersystemen des Standes der Technik erhöht ist, aber gleichzeitig die Viskosität des Blends während der Verarbeitung so gestaltet ist, dass ein Ausfüllen einer Form und gegebenenfalls ein Durchtränken von vorhandenem Fasermaterial noch möglich ist.

EP-A 1358997 beschreibt ein Verfahren zur Herstellung von Autoreifen, wobei zur Aushärtung der Reifen keine Epoxide eingesetzt werden. Der Einsatz von einem Guanidin-Derivat in Gegenwart von anderen Aminhärterkomponenten mit einer Funktionalität ≥ 2 für ein Epoxidharz wird jedoch nicht offenbart. Des Weiteren offenbart EP-A 1358997 auch nicht, dass ein Blend, das die Härterkomponenten a) und b) enthält und in den beschriebenen Mengenverhältnissen zueinander vorliegt, so steuerbar ist, dass die Aushärtegeschwindigkeit (Topfzeit) des Blends gegenüber den Härtersystemen des Standes der Technik erhöht ist, aber gleichzeitig die Viskosität des Blends während der Verarbeitung so gestaltet ist, dass ein Ausfüllen einer Form und gegebenenfalls ein Durchtränken von vorhandenem Fasermaterial noch möglich ist

WO-A 03/045801 beschreibt einen Plastikkontainer, der unter anderem auch ein Epoxidharz enthält, das mit Hilfe von Härtern gehärtet werden kann. Unter den in WO-A 03/045801 genannten Härtern sind unter anderem auch primäre und sekundäre Amine aber auch Guanidin-Derivate wie TMG oder Dicyandiamin genannt. Des Weiteren beschreibt diese Druckschrift, dass das Verhältnis von Epoxidäquivaleten zu thermisch härtbaren Äquivalenten im Bereich von 0,9:1 bis 2:1 sein soll. Den Einsatz einer Mischung aus Härterkomponenten a) und b) mit einem Anteil der Guanidin-Derivate im Bereich von 5 bis 55 Gew.-% bezogen auf die Mischung wird jedoch nicht offenbart. WO-A 03/045801 beschreibt des Weiteren nicht, dass ein Blend, das die Härterkomponenten a) und b) enthält und in den beschriebenen Mengenverhältnissen zueinander vorliegt, so steuerbar ist, dass die Aushärtegeschwindigkeit (Topfzeit) des Blends gegenüber den Härtersystemen des Standes der Technik erhöht ist, aber gleichzeitig die Viskosität des Blends während der Verarbeitung so gestaltet ist, dass ein Ausfüllen einer Form und gegebenenfalls ein Durchtränken von vorhandenem Fasermaterial noch möglich ist.

WO-A 03/002667 beschreibt eine härtbare Mischung, die unter anderem Epoxidharze enthält. Des Weiteren beschreibt WO-A 03/002667 den Einsatz von Härter in denen auch Amine mit einer Funktionalität ≥ 2 und Guanidin-Derivate der Formel I genannt sind sowie Mischungen dieser Härter. WO-A 03/002667 beschreibt jedoch nicht den Einsatz von Härterkomponenten a) und b) und ihre jeweiligen Verhältnisse zu einander. WO-A 03/002667 beschreibt des Weiteren nicht, dass ein Blend, das die Härterkomponenten a) und b) enthält und in den beschriebenen Mengenverhältnissen zueinander vorliegt, so steuerbar ist, dass die Aushärtegeschwindigkeit (Topfzeit) des Blends gegenüber den Härtersystemen des Standes der Technik erhöht ist, aber gleichzeitig die Viskosität des Blends während der Verarbeitung so gestaltet ist, dass ein Ausfüllen einer Form und gegebenenfalls ein Durchtränken von vorhandenem Fasermaterial noch möglich ist.

GB-A 2003-213909 und JP-A 2002/240205 beschreiben wie auch GB-A 2005-246390 sowie JP-A 2005/067209 eine Polyolefinharzfolie, die neben Polyolefinschichten auch eine Schicht aus Textilfaser enthält, wobei die Polyolefinschichten aus unterschiedlichen Polyolefinmischungen und nicht-flammbaren Partikeln bestehen. Die nicht-flammbaren Partikel werden mit Hilfe von aushärtbaren Harzen auf die Polyolefinschichten aufgeklebt. Alle Dokumente beschreiben weder den Einsatz eines Blends aus den beiden Härterkomponenten a) und b) noch deren Mengenverhältnisse zueinander. Des Weiteren wird auch in GB-A 2003-213909 und JP-A 2002/240205 nicht beschrieben, dass ein Blend, das die Härterkomponenten a) und b) enthält und in den beschriebenen Mengenverhältnissen zueinander vorliegt, so steuerbar ist, dass die Aushärtegeschwindigkeit (Topfzeit) des Blends gegenüber den Härtersystemen des Standes der Technik erhöht ist, aber gleichzeitig die Viskosität des Blends während der Verarbeitung so gestaltet ist, dass ein Ausfüllen einer Form und gegebenenfalls ein Durchtränken von vorhandenem Fasermaterial noch möglich ist.

WO-A 021070623 beschreibt die Herstellung eines Fluorpolymerfilms, der einen Klebstoff auf einer Seite des Films aufweist. Dieser Klebstoff, soll ein thermisch härtbarer Klebstoff sein, der Epoxidharze enthält. Diese Epoxidharze enthalten weiterhin Härterkomponenten, wobei unter anderem auch Polyetheramine und Guanidin-Derivate der Formel I genannt sind. Eine Härtermischung aus Härterkomponente a) und b) mit einem einem Anteil der Guanidin-Derivate im Bereich von 5 bis 55 Gew.-% bezogen auf die Mischung wird jedoch nicht offenbart. WO-A 02/070623 beschreibt des Weiteren nicht, dass ein Blend, das die Härterkomponenten a) und b) enthält und in den beschriebenen Mengenverhältnissen zueinander vorliegt, so steuerbar ist, dass die Aushärtegeschwindigkeit (Topfzeit) des Blends gegenüber den Härtersystemen des Standes der Technik erhöht ist, aber gleichzeitig die Viskosität des Blends während der Verarbeitung so gestaltet ist, dass ein Ausfüllen einer Form und gegebenenfalls ein Durchtränken von vorhandenem Fasermaterial noch möglich ist.

Die Aufgabe der vorliegenden Erfindung ist daher ein Blend bereit zustellen, das es ermöglicht, die Aushärtungsgeschwindigkeit des Blends zu erhöhen ohne gleichzeitig die Viskosität des Blends während der Verarbeitung so zu erhöhen, dass eine vollständig Ausfüllung der Form und gegebenenfalls gleichmäßige Durchtränkung von vorhandenem Fasermaterial, nicht mehr möglich ist.

Diese Aufgabe wird gelöst durch ein Blend enthaltend
α) ein oder mehrere Epoxidharze und
β) eine Mischung enthaltend
   1) 0,3 bis 0,9 Aminäquivaiente, pro Äquivalent Epoxid der eingesetzten Komponente α), an einer Härterkomponente a) und
   2) eine Härterkomponente b),
      dadurch gekennzeichnet, dass die Härterkomponente a) ein oder mehrere Amine mit einer Funktionalität ≥ 2 enthält und mindestens ein Amin beim stöchiometrischen Vermischen mit dem Epoxidharz im 100 g Ansatz bei Raumtemperatur zu einer Aushärtungszeit von weniger als 24 h führt und die Härterkomponente b) mindestens eine Verbindung der Formel I 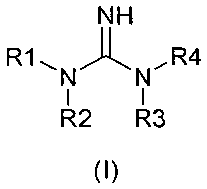
enthält, wobei R1 bis R3, R5 und R6 unabhängig voneinander einen organischen Rest mit 1 bis 20 C-Atomen und Wasserstoff bedeutet und R4 ausgewählt ist aus der Gruppe von einem organischen Rest mit 1 bis 20 C-Atomen und einer -C(NH)NR5R6 Gruppe.

Vorteilhaft ist das erfindungsgemäße Blend, dadurch gekennzeichnet, dass die Härterkomponente a) ausgewählt ist aus der Gruppe von Aminen mit einer Funktionalität ≥ 2.

Vorteilhaft ist das erfindungsgemäße Blend, dadurch gekennzeichnet, dass, die Härterkomponente a) mindestens zwei Härterkomponenten a1) und a2) enthält, wobei die Härterkomponente a1) mindestens ein Polyetheramine mit einer Funktionalität ≥ 2 ist und die Härterkomponente a2) mindestens ein weiteres Amin mit einer Funktionalität ≥ 2 ist.

Vorteilhaft ist das erfindungsgemäße Blend, dadurch gekennzeichnet, dass
die Härterkomponente a1) ausgewählt ist aus der Gruppe von 3,6-dioxa-1,8-octandiamin, 4,7,10-Trioxa-1,13-tridecandiamin, 4,7-Dioxa-1,10-decandiamin, 4,9-Dioxa-1,12-docecandiamin, Polyetheramin auf der Basis von Triethylenglykol mit einer mittleren Molmasse von 148, difunktionellem, primären Polyetheramin hergestellt durch Aminierung eines mit Propylenoxid aufgepfropften Ethylenglykols mit einer mittleren Molmasse von 176, difunktionellem, primären Polyetheramin auf der Basis von Propylenoxid mit einer mittleren Molmasse von 4000, difunktionellem, primären Polyetheramin hergestellt durch Aminierung eines mit Propylenoxid aufgepfropften Polyethylenglykols mit einer mittleren Molmasse von 2003, aliphatischem Polyetheramin auf der Basis von mit Propylenoxid gepfropftem Polyethylengylkol mit einer mittleren Molmasse von 900, aliphatischem Polyetheramin auf der Basis von mit Propylenoxid gepfropftem Polyethylenglykol mit einer mittleren Molmasse von 600, difunktionellem, primären Polyetheramin hergestellt durch Aminierung eines mit Propylenoxid aufgepfropften Diethylenglykols mit einer mittleren Molmasse von 220, aliphatischem Polyetheramin auf der Basis eines Copolymers aus Poly(tetramethylen-etherglycol) und Polypropylenglycol mit einer mittleren Molmasse von 1000, aliphatischem Polyetheramin auf der Basis eines Copolymers aus Poly(tetramethylenetherglycol) und Polypropylenglycol mit einer mittleren Molmasse von 1900, aliphatischem Polyetheramin auf der Basis eines Copolymers aus Poly(tetramethylenetherglycol) und Polypropylenglycol mit einer mittleren Molmasse von 1400, Polyethertriamin auf der Basis eines mit Butylenoxid gepfropftem mindestens dreiwertigen Alkohols mit einer mittleren Molmasse von 400, aliphatischem Polyetheramin hergestellt durch Aminierung von mit Butylenoxid aufgepfropften Alkoholen mit einer mittleren Molmasse von 219, Polyetheramin auf der Basis von Pentaerythrit und Propylenoxid mit einer mittleren Molmasse von 600, difunktionellem, primären Polyetheramin auf Basis von Polypropylenglykol mit einer mittleren Molmasse von 2000, difunktionellem, primären Polyetheramin auf der Basis von Polypropylenglykol mit einer mittleren Molmasse von 230, difunktionellem, primären Polyetheramin auf der Basis von Polypropylenglykol mit einer mittleren Molmasse von 400, trifunktionellem, primären Polyetheramin hergestellt durch Reaktion von Propylenoxid mit Trimethylolpropan gefolgt durch eine Aminierung der endständigen OH-Gruppen mit einer mittleren Molmasse von 403, trifunktionellem, primären Polyetheramin hergestellt durch Reaktion von Propylenoxid mit Glycerin gefolgt durch eine Aminierung der endständigen OH-Gruppen mit einer mittleren Molmasse von 5000 und einem Polyetheramin mit einer mittleren Molmasse von 400 hergestellt durch Aminierung von PolyTHF, das eine mittlere Molmasse von 250 aufweist und
die Härterkomponente a2) ausgewählt aus der Gruppe von 1,12-Diaminododecan, 1,10-Diaminodecan, 1,2-Diaminocyclohexan, 1,2-Propandiamin, 1,3-Bis(aminomethyl)cyclohexan, 1,3-Propandiamin, 1- Methyl-2,4-diaminocyclohexan, 2,2'-Oxy-bis(ethylamin), 3,3'-Dimethyl-4,4'-diaminodicyclohexylmethan, 4,4'-Methylendianilin, 4-Ethyl-4-methylamino-1-octylamin, Diethylentriamin, Ethylendiamin, Hexamethylendiamin, Isophorondiamin, Menthendiamin, Xylylendiamin, N-Aminoethylpiperazin, Neopentandiamin, Norbornandiamin, Octanmethylendiamin, Piperazin, 4,8-Diamino-tri-cyclo[5.2.1.0]decan, Tolylendiamin, Triethylentetramin und Trimethylhexamethylendiamin ist.

Vorteilhaft ist das erfindungsgemäße Blend, dadurch gekennzeichnet, dass die Reste R1 bis R3, R5 und R6 der Verbindungen der Formel I unabhängig voneinander ausgewählt sind aus der Gruppe von Wasserstoff, Methyl, Ethyl, n-Propyl, i-Propyl, n-Butyl, sec.- Butyl, Phenyl und o-Tolyl und R4 ausgewählt ist aus der Gruppe von Methyl, Ethyl, n-Propyl, i-Propyl, n-Butyl, sec.- Butyl, Phenyl, o-Tolyl und einer -C(NH)NR5R6-Gruppe.

Vorteilhaft ist das erfindungsgemäße Blend, dadurch gekennzeichnet, dass der Anteil an Härterkomponente b) bezogen auf den Gewichtsanteil der Mischung 5 bis 55 Gew.-% beträgt.

Vorteilhaft ist das erfindungsgemäße Blend, dadurch gekennzeichnet, dass die Mischung als Härterkomponente a1) ein Polyetheramin mit einer Funktionalität von ≥ 2 ausgewählt aus der Gruppe von difunktionellem, primären Polyetheramin auf der Basis von Polypropylenglykol mit einer mittleren Molmasse von 230, difunktionellem, primären Polyetheramin auf der Basis von Polypropylenglykol mit einer mittleren Molmasse von 400, aliphatischem, difunktionellem, primären Polyetheramin auf Basis von Polypropylenglykol mit einer mittleren Molmasse von 2000, difunktionellem, primären Polyetheramin hergestellt durch Aminierung eines mit Propylenoxid aufgepfropften Diethylenglykols mit einer mittleren Molmasse von 220, trifunktionellem, primären Polyetheramin hergestellt durch Reaktion von Propylenoxid mit Trimethylolpropan gefolgt durch eine Aminierung der endständigen OH-Gruppen mit einer mittleren Molmasse von 403, aliphatischem Polyetheramin auf der Basis von mit Propylenoxid gepfropftem Polyethylengylkol mit einer mittleren Molmasse von 900, aliphatischem Polyetheramin auf der Basis eines Copolymers aus Poly(tetramethylenetherglycol) und Polypropylenglycol mit einer mittleren Molmasse von 1000, aliphatischem Polyetheramin auf der Basis eines Copolymers aus Poly(tetramethylenetherglycol) und Polypropylenglycol mit einer mittleren Molmasse von 1900, aliphatischem Polyetheramin auf der Basis eines Copolymers aus Poly(tetramethylenetherglycol) und Polypropylenglycol mit einer mittleren Molmasse von 1400, Polyethertriamin auf der Basis eines mit Butylenoxid gepfropftem mindestens dreiwertigen Alkohols mit einer mittleren Molmasse von 400, aliphatischem Polyetheramin hergestellt durch Aminierung von mit Butylenoxid aufgepfropftem Alkoholen mit einer mittleren Molmasse von 219, trifunktionellem, primären Polyetheramin hergestellt durch Reaktion von Propylenoxid mit Glycerin gefolgt durch eine Aminierung der endständigen OH-Gruppen mit einer mittleren Molmasse von 5000 und als Härterkomponente a2) ein weiteres Amin mit einer Funktionalität ≥ 2 ausgewählt aus der Gruppe von Isophorondiamin, Aminoethylpiperazin, 1,3 Bis(aminoethyl)cyclohexan, Triethylentetramin, wobei das Verhältnis von a1) zu a2) im Bereich von 0,1 bis 10 zu 1 liegt

Vorteilhaft ist das erfindungsgemäße Blend, dadurch gekennzeichnet, dass die Mischung als Härterkomponente a1) ein Polyetheramin ausgewählt aus der Gruppe von Polyetheramin D 230, Polyetheramin D 400, Polyetheramin T 403, Polyetheramin T 5000, die Härterkomponente a2) ausgewählt ist aus der Gruppe von Isophorondiamin, Aminoethylpiperazin, 1,3-Bis(aminomethyl)cyclohexan und Triethylentetraamin und die Härterkomponente b) Tetramethylguanidin ist und das Verhältnis von Härterkomponente a1) zu Härterkomponente a2) im Bereich von 1,5 bis 10 zu 1 liegt und 10 bis 60 Mol % weniger an Mischung zum Epoxidharz gegeben wird als zur Umsetzung der aktiven Epoxygruppen an Aminfunktionen der Mischung benötigt werden.

Vorteilhaft ist das erfindungsgemäße Blend, dadurch gekennzeichnet, dass das Blend noch faserverstärktes Material enthält.

Ein weiterer Gegenstand der Erfindung ist ein Verfahren zur Herstellung des erfindungsgemäßen Blends, dadurch gekennzeichnet, dass ein oder mehrere Epoxidharze mit der Mischung bei Temperaturen unterhalb der Anfangshärtungstemperatur der Härterkomponente a) vermischt werden.

Ein weiterer Gegenstand der Erfindung ist die Verwendung des erfindungsgemäßen Blends zur Herstellung von gehärteten Epoxidharzen.

Vorteilhaft ist die erfindungsgemäße Verwendung, dadurch gekennzeichnet, dass die gehärteten Epoxidharze Formkörper sind.

Vorteilhaft ist die erfindungsgemäße Verwendung, dadurch gekennzeichnet, dass die Formkörper verstärkendes Material enthalten.

Ein weiterer Gegenstand der Erfindung ist ein gehärtetes Epoxidharz erhältlich durch Härtung des erfindungsgemäßen Blends.

Vorteilhaft ist das erfindungsgemäße gehärtete Epoxidharz, dadurch gekennzeichnet, dass das gehärtete Epoxidharz einen Formkörper darstellt.

Ein weiterer Gegenstand der Erfindung ist ein Formkörper, dadurch gekennzeichnet dass er faserverstärkt ist.

Vorteilhaft ist der erfindungsgemäße Formkörper, erhältlich durch Aushärtung einer Form die mit einem faserverstärktem Material ausgelegt ist und Einbringung des erfindungsgemäßen Blends mittels Infusionstechnologie in die Form.

Vorteilhaft ist der erfindungsgemäße Formkörper, dadurch gekennzeichnet, dass der Formkörper verstärkte Bauteile für Rotorblätter von Windkraftanlagen darstellt.

Die erfindungsgemäßen Blends, enthalten mindestens ein und/oder mehrere Epoxidharze und eine Mischung aus einer Härterkomponente a) und einer Härterkomponente b). Die zu verwendenden Epoxidharze und/oder Epoxidharzmischungen enthalten dabei bevorzugt Epoxidharze ausgewählt aus der Gruppe von Bisphenol-A-bisglycidylether (DGEBA), Bisphenol-F-bisglycidylether, Bisphenol-S-bisglycidether (DGEBS), Tetraglycidylmethylendianiline (TGMDA), Epoxy-Novolaken (den Reaktionsprodukten aus Epichlorhydrin und Phenolharzen (Novolak)) und cycloaliphatischen Epoxidharzen wie 3,4-Epoxycyclohexylmethyl-3,4-epoxycyclohexancarboxylat und Hexahydrophthalsäurediglycidylester.

Des Weiteren können die Epoxidharze auch noch weitere Raktivverdünner enthalten. Diese sind ausgewählt aus der Gruppe von 1,4-Butandiolbisglycidylether, 1,6-Hexandiolbisglycidylether, Glycidylneodecanoat, Glycidylversatat, 2-Ethylhexylglycidylether, C₈-C₁₀ - Alkylglycidylether, C₁₂-C₁₄ - Alkylglycidylether, p-tert-Butylglycidether, Butylglycidether, Nonylphenylglycidether, p-tert-Butylphenylglycid-ether, Phenylglycidether, o-Cresylglycidether, Polyoxypropylenglycoldiglycidether, Trimethylolpropantriglycidether (TMP), Glycerintriglycidether und Triglycidylparaaminophenol (TGPAP).

Gemäß dem Stand der Technik wird zum Aushärten von Epoxidharzen eine nahezu stöchiometrische Menge (je nach Epoxidharz 0,9 - 1,1 Äquivalente des Härters / Äquivalent Epoxidharz) eingesetzt. Wenn jedoch die erfindungsgemäße Mischung zum Härten von Epoxidharzen eingesetzt wird, ist es bevorzugt, dass man 10 bis 60 Mol-%, besonders bevorzugt 20 bis 40 Mol-% weniger der erfindungsgemäßen Mischung zum Epoxidharz gibt, als zur Umsetzung der aktiven Epoxygruppen an Aminfunktionen der Mischung benötigt werden. Besonders bevorzugt ist, wenn man in der Summe 0,3 bis 0,9 Aminäquivalent, bevorzugt 0,4 bis 0,7 Aminäquivalent, pro Äquivalent Epoxid des eingesetzten Epoxidharzes, an Härterkomponenten a1) und a2) zur Mischung gibt, um eine Erhöhung der Topfzeit und eine vergleichbare bis bessere Aushärtung des Epoxidharzes gegenüber den zum Stand der Technik gehörenden Mischungen zu erzielen. Für das erfindungsgemäße Blend beträgt der Anteil der Härterkomponente a) 0,3 bis 0,9, bevorzugt 0,4 bis 0,7 Aminäquivalent, pro Äquivalent Epoxid des eingesetzten Epoxidharzes.

Zur Herstellung des erfindungsgemäßen Blends und für das erfindungsgemäße Verfahren wird die Mischung mit dem Epoxidharz bei Temperaturen die unterhalb der Anfangshärtungstemperatur der Härterkomponente a) liegt, vermischt. Die Anfangshärtungstemperatur ist die Temperatur bei der in einer Mischung von mehreren Härterkomponenten mit einer Funktionalität ≥ 2 die erste Härterkomponente mit dem Epoxidharz reagiert. Dieser Temperatur kann mit einer DSC nach DIN 53765 als T_{RO}^{E} ermittelt werden.

Die Härterkomponente a) im erfindungsgemäßen Blend, sowie für das erfindungsgemäße Verfahren enthält dabei ein oder mehrere Amine mit einer Funktionalität ≥ 2, wobei mindestens ein Amin beim stöchiometrischen Vermischen mit dem Epoxidharz im 100 g Ansatz bei Raumtemperatur zu einer Aushärtungszeit von weniger als 24 h führt.
Die Amine mit einer Funktionalität ≥ 2 der Härterkomponente a) sind alle dem Fachmann bekannten Amine mit einer Funktionalität ≥ 2. Bevorzugt sind diese ausgewählt aus der Gruppe von 3,6-Dioxa-1,8-octandiamin, 4,7,10-Trioxa-1,13-tridecandiamin, 4,7-Dioxa-1,10-decandiamin, 4,9-Dioxa-1,12-docecandiamin, Polyetheramin auf der Basis von Triethylenglykol mit einer mittleren Molmasse von 148, difunktionellem, primären Polyetheramin hergestellt durch Aminierung eines mit Propylenoxid aufgepfropftem Ethylenglykols, mit einer mittleren Molmasse von176, difunktionellem, primären Polyetheramin auf der Basis von Propylenoxid mit einer mittleren Molmasse von 4000, difunktionellem, primären Polyetheramin hergestellt durch Aminierung eines mit Propylenoxid aufgepfropftem Polyethylenglykols mit einer mittleren Molmasse von 2003, aliphatischem Polyetheramin auf der Basis von mit Propylenoxid gepfropftem Polyethylengylkol mit einer mittleren Molmasse von 900, aliphatischem Polyetheramin auf der Basis von mit Propylenoxid gepfropftem Polyethylenglykol mit einer mittleren Molmasse von 600, difunktionellem, primären Polyetheramin hergestellt durch Aminierung eines mit Propylenoxid aufgepfropftem Diethylenglykols mit einer mittleren Molmasse von 220, aliphatischem Polyetheramin auf der Basis eines Copolymers aus Poly(tetramethylenetherglycol) und Polypropylenglycol mit einer mittleren Molmasse von 1000, aliphatischem Polyetheramin auf der Basis eines Copolymers aus Poly(tetramethylenetherglycol) und Polypropylenglycol mit einer mittleren Molmasse von 1900, aliphatischem Polyetheramin auf der Basis eines Copolymers aus Poly(tetramethylenetherglycol) und Polypropylenglycol mit einer mittleren Molmasse von 1400, Polyethertriamin auf der Basis eines mit Butylenoxid gepfropftem mindestens dreiwertigen Alkohols mit einer mittleren Molmasse von 400, aliphatischem Polyetheramin hergestellt durch Aminierung von mit Butylenoxid aufgepfropftem Alkoholen mit einer mittleren Molmasse von 219, Polyetheramin auf der Basis von Pentaerythrit und Propylenoxid mit einer mittleren Molmasse von 600, difunktionellem, primären Polyetheramin auf Basis von Polypropylenglykol mit einer mittleren Molmasse von 2000, difunktionellem, primären Polyetheramin auf der Basis von Polypropylenglykol mit einer mittleren Molmasse von 230, difunktionellem, primären Polyetheramin auf der Basis von Polypropylenglykol mit einer mittleren Molmasse von 400, trifunktionellem, primärem Polyetheramin hergestellt durch Reaktion von Propylenoxid mit Trimethylolpropan gefolgt durch eine Aminierung der endständigen OH-Gruppen mit einer mittleren Molmasse von 403, trifunktionellem, primären Polyetheramin hergestellt durch Reaktion von Propylenoxid mit Glycerin gefolgt durch eine Aminierung der endständigen OH-Gruppen mit einer mittleren Molmasse von 5000, Polyetheramin mit einer mittleren Molmasse von 400, hergestellt durch Aminierung von PolyTHF, das eine mittlere Molmasse von 250 aufweist,1,12-Diaminododecan, 1,10-Diaminodecan, 1,2-Diaminocyclohexan, 1,2-Propandiamin, 1,3-Bis(aminomethyl)cyclohexan, 1,3-Propandiamin, 1- Methyl-2,4-diaminocyclohexan, 2,2'-Oxybis(ethylamin), 3,3'-Dimethyl-4,4'-diaminodicyclohexylmethan, 4-Ethyl-4-methylamino-1-octylamin, Diethylentriamin, Ethylendiamin, Hexamethylendiamin, Isophorondiamin, Menthendiamin, Xylylendiamin, N-Aminoethylpiperazin, Neopentandiamin, Norbornandiamin, Octanmethylendiamin, Piperazin, 4,8-Diamino-tricyclo[5.2.1.0]decan, Tolylendiamin, Triethylentetramin und Trimethylhexamethylendiamin.

Besonders bevorzugt enthält die Härterkomponente a) mindestens zwei Härterkomponenten a1) und a2), wobei beide ein Amin mit einer Funktionalität ≥ 2 enthalten. Ganz besonders bevorzugt enthält die Härterkomponente a1) ein Polyetheramin und die Härterkomponente a2) ein weiteres Amin mit einer Funktionalität ≥ 2.

Polyamine, in deren Kette Sauerstoff enthalten ist, werden als Polyetheramine bezeichnet. Polyetheramine mit einer Funktionalität von ≥ 2 können in dem erfindungsgemäßen Blend und dem erfindungsgemäßen Verfahren als Härterkomponente a) und in der erfindungsgemäßen Mischung als Härterkomponente a1) eingesetzt werden. Sie können u.a. auf Basis von Alkylenoxiden wie Ethylen-, Propylen-, Butylen oder Pentylenoxid, Poly-THF oder 1,4-Butandiol und jeweils Ammoniak hergestellt werden und weisen Molgewichtsverteilungen auf. Die eingesetzten Alkylenoxide können gleich oder unterschiedlich pro Molekül sein. Bei den Polyetheraminen des D, ED und EDR-Typs handelt es sich um Diamine (D-Typ), wobei ED für Diamine auf Basis Polyethylenglykol (PEG) und EDR für reaktive Diamine auf Basis PEG steht. bei den T-Typen handelt es sich um ein mit Alkylenoxid(en) gepfropftes Triol, das an den drei Termini jeweils eine Aminogruppe trägt. XTJ wird für noch zur Erprobung vorgesehene Produkte verwendet. Die hinter dem Buchstabencode stehenden Zahlen, außer bei den XTJ Produkten, in der Bezeichnung der Polyetheramine, gibt die mittlere Molmasse des Polyetheramins wieder.
Die in der erfindungsgemäßen Mischung, dem erfindungsgemäßem Blend und dem erfindungsgemäßen Verfahren eingesetzten Polyetheramine weisen eine Funktionalität von ≥ 2 auf.
Typische Beispiele für Polyetheramine der Härterkomponente a1) sind ausgewählt aus der Gruppe von difunktionellem, primären Polyetheramin auf der Basis von Polypropylenglykol mit einer mittleren Molmasse von 230, difunktionellem, primären Polyetheramin auf der Basis von Polypropylenglykol mit einer mittleren Molmasse von 400, difunktionellem, primären Polyetheramin auf Basis von Polypropylenglykol mit einer mittleren Molmasse von 2000, difunktionellem, primären Polyetheramine auf der Basis von Propylenoxid mit einer mittleren Molmasse von 4000, trifunktionellem, primären Polyetheramin hergestellt durch Reaktion von Propylenoxid mit Trimethylolpropan gefolgt durch eine Aminierung der endständigen OH-Gruppen mit einer mittleren Molmasse von 403, trifunktionellem, primären Polyetheramin hergestellt durch Reaktion von Propylenoxid mit Glycerin gefolgt durch eine Aminierung der endständigen OH-Gruppen mit einer mittleren Molmasse von 5000. Diese Verbindungen sind auch Verkaufsprodukte der Firma BASF (Polyetheramine) und der Firma Huntsman (Jeffamine) und sind unter folgenden Handelsnamen erhältlich:

Polyetheramine D 230 / Jeffamine^{®} D 230:
enthält Polyetheramin auf der Basis von Polypropylenglykol mit einer mittleren Molmasse von 230.

Polyetheramine D 400 / Jeffamine^{®} XTJ 582:
enthält difunktionelles, primäres Polyetheramin auf der Basis von Polypropylenglykol mit einer mittleren Molmasse von 400.

Polyetheramine D 2000 / Jeffamine^{®} D2000 / Jeffamine^{®} XTJ 578:
enthält aliphatisches, difunktionelles, primäres Polyetheramin auf Basis von Polypropylenglykol mit einer mittleren Molmasse von 2000.

Polyetheramine D 4000:
enthält Polyetheramine auf der Basis von Polypropylenglykol mit einer mittleren Molmasse von 4000.

Polyetheramine T 403 / Jeffamine^{®} T 403:
enthält Polyetheramin hergestellt durch Reaktion von Propylenoxid mit Trimethylolpropan gefolgt durch eine Aminierung der endständigen OH-Gruppen mit einer mittleren Molmasse von 403.

Polyetheramine T 5000 / Jeffamine^{®} T 5000:
enthält Polyetheramin hergestellt durch Reaktion von Propylenoxid mit Glycerin gefolgt durch eine Aminierung der endständigen OH-Gruppen mit einer mittleren Molmasse von 5000.

Jeffamine^{®} ED-600 / Jeffamine^{®} XTJ 501:
enthält ein aliphatisches Polyetheramin, das aus einem mit Propylenoxid gepfropftem Polyethylenglykol aufgebaut ist und eine mittlere Molmasse von 600 aufweist.

Jeffamine^{®} ED-900:
enthält ein aliphatisches Polyetheramin, das aus einem mit Propylenoxid gepfropftem Polyethylenglykol aufgebaut ist und eine mittlere Molmasse von 900 aufweist.

Jeffamine^{®} ED-2003:
enthält ein aliphatisches Polyetheramin, das aus einem mit Propylenoxid gepfropftem Polyethylenglykol aufgebaut ist und eine mittlere Molmasse von 2000 aufweist.

Jeffamine^{®} HK-511:
enthält ein difunktionelles, primäres Polyetheramin hergestellt durch Aminierung eines mit Propylenoxid gepfropftem Diethylenglykols mit einer mittleren Molmasse von 220.

Jeffamine^{®} XTJ-542:
enthält ein aliphatisches Polyetheramin auf der Basis eines Copolymers aus Poly(tetramethylenetherglycol) und Polypropylenglycol mit einer mittleren Molmasse von 1000.

Jeffamine^{®} XTJ-548:
enthält ein aliphatisches Polyetheramin auf der Basis eines Copolymers aus Poly(tetramethylenetherglycol) und Polypropylenglycol mit einer mittleren Molmasse von 1900.

Jeffamine^{®} XTJ-559:
enthält Copolymers aus Poly(tetramethylenetherglycol) und Polypropylenglycol mit einer mittleren Molmasse von 1400.

Jeffamine^{®} XTJ-566:
enthält Polyethertriamin auf der Basis eines mit Butylenoxid gepfropftem mindestens dreiwertigen Alkohols mit einer mittleren Molmasse von 400.

Jeffamine^{®} XTJ-568:
enthält ein aliphatisches Polyetheramin hergestellt durch Aminierung von mit Butylenoxid aufgepfropften Alkoholen mit einer mittleren Molmasse von 219.

Jeffamine^{®} XTJ- 616:
enthält ein Polyetheramin auf der Basis von Pentaerythrit und Propylenoxid mit einer mittleren Molmasse von 600.

Jeffamine^{®} EDR-148:
enthält ein Polyetheramine auf der Basis von Triethylenglykol mit einer mittleren Molmasse von 148.

Jeffamine^{®} EDR-176:
enthält ein difunktionelles, primäres Polyetheramin hergestellt durch Aminierung eines mit Propylenoxid gepfropften Ethylenglykols, mit einer mittleren Molmasse von 176.

PolyTHF-Amin 350:
enthält ein Polyetheramin hergestellt durch Aminierung von PolyTHF mit einer mittleren Mollmasse von 250. Das dadurch erhaltene PolyTHF-Amin besitzt ein mittleres Molekulargewicht von 400.

Die Polyetheramine der Härterkomponente a1) sind bevorzugt ausgewählt aus der Gruppe von difunktionellem, primären Polyetheramin hergestellt durch Aminierung eines mit Propylenoxid aufgepfropftem Diethylenglykols mit einer mittleren Molmasse von 220, aliphatischem Polyetheramin auf der Basis von mit Propylenoxid gepfropftem Polyethylengylkol mit einer mittleren Molmasse von 900, aliphatischem Polyetheramin auf der Basis eines Copolymers aus Poly(tetramethylenetherglycol) und Polypropylenglycol mit einer mittleren Molmasse von 1000, aliphatischem Polyetheramin auf der Basis eines Copolymers aus Poly(tetramethylenetherglycol) und Polypropylenglycol mit einer mittleren Molmasse von 1900, aliphatischem Polyetheramin auf der Basis eines Copolymers aus Poly(tetra-methylenetherglycol) und Polypropylenglycol mit einer mittleren Molmasse von 1400,
Polyethertriamin auf der Basis eines mit Butylenoxid gepfropftem mindestens dreiwertigen Alkohols mit einer mittleren Molmasse von 400, aliphatischem Polyetheramin hergestellt durch Aminierung von Butylenoxid gepfropften Alkoholen mit einer mittleren Molmasse von 219, difunktionellem, primären Polyetheramin auf der Basis von Polypropylenglykol mit einer mittleren Molmasse von 230, difunktionellem, primären Polyetheramin auf der Basis von Polypropylenglykol mit einer mittleren Molmasse von 400, trifunktionellem, primären Polyetheramin hergestellt durch Reaktion von Propylenoxid mit Trimethylolpropan gefolgt durch eine Aminierung der endständigen OH-Gruppen mit einer mittleren Molmasse von 403 und Polyetheramin auf Basis von Propylenoxid und Glycerin mit einer mittleren Molmasse von 5000. Ganz besonders bevorzugt ist als Polyetheramin ein Polyetheramin auf der Basis von Polypropylenglykol mit einer mittleren Molmasse von 230 wie z.B. Polyetheramine D 230 oder Jeffamine^{®} D230.

Als Härterkomponente a2) sind weitere Amine mit einer Funktionalität ≥ 2 ausgewählt aus der Gruppe von 1,12-Diaminododecan, 1,10-Diaminodecane, 1,2-Diaminocyclohexan, 1,2-Propandiamin, 1,3-Bis(aminomethyl)cyclohexan, 1,3-Propandiamin, 1-Methyl-2,4-diaminocyclohexan, 2,2'-Oxybis(ethylamine), 3,3'-Dimethyl-4,4'-diaminodicyclohexylmethan, 4,4'-Methylendianilin, 4-Ethyl-4-methylamino-1-octylamin, Diethylentriamin, Ethylendiamin, Hexamethylendiamin, Isophorondiamin, Menthendiamin, Xylylendiamin, N-Aminoethylpiperazin, Neopentandiamin, Norbornandiamin, Octanmethylendiamin, Piperazin 4,8-Diamino-tricyclo[5.2.1.0]decan,Tolylendiamin, Triethylentetramin, Trimethylhexamethylendiamin,

In der erfindungsgemäßen Mischung, dem erfindungsgemäßen Blend sowie im erfindungsgemäßen Verfahren können auch noch Beschleuniger enthalten sein. Diese sind ausgewählt aus der Gruppe von substituierten Imidazolen wie 1-Methylimidazol, 2 Methylimidazol, 2,4-Ethyl-Methyl-imidazol, 2-Phenylimidazol, 1-Cyanoethylimidazol, Imidazolinen wie 2-Phenyl-imidazolin, tertiären Aminen wie N,N-Dimethyl-benzylamin; 2,4,6-Tris-(dimethylaminomethyl)-phenol (DMP 30), Bisphenol-A, Bisphenol-F, Nonylphenol, p-tert-Butylphenol, Phenolharze des Novolak-Typs, Salicylsäure, p-Toluolsulfonsäure,1,4-Diazabicyclo[2,2,2]octan (DABCO), 1,8-Diazabicyclo-[5.4.0]-undecen-7 (DBU), S-Triazin (Lupragen N 600), Bis-(2-dimethylaminoethyl)ether (Lupragen N 206), Pentamethyldiethylentriamin (Lupragen N 301), Trimethylaminoethylethanolamin (Lupragen N 400), Tetramethyl-1,6-hexandiamin (Lupragen N 500), Aminoethylmorpholin, Aminopropylmorpholin, Aminoethylethylenharnstoff, Ketiminen wie Epi-Kure 3502 (ein Reaktionsprodukt aus Ethylenediamin mit Methylisobutylketon), Uronen wie 3-(4-chlorophenyl)-1,1-dimethyl-harnstoff (Monuron), 3-(3,4-dichlorophenyl)-1,1-dimethyl-harnstoff (Diuron), 3-phenyl-1,1-dimethylharnstoff (Fenuron), 3-(3-Chloro-4-methylphenyl)-1,1-dimethylharnstoff (Chlorotoluron), Tolyl-2,4 bis-N,N-dimethyl-carbamid (Amicure UR2T), Dicyandiamid (DICY), Mannich-Basen oder sekundären Aminen wie Dialkylaminen wie beispielsweise Di-(2-ethylhexyl)amin, Dibutylamin, Dipropylamin, Ditridecylamin, N, N'-Diisopropylisophorondiamin (Jefflink^{®} XTJ-584), N,N'-Diisobutyl-4,4'-Diamino-dicyclohexylmethan (Clearlink 1000), N-(Hydroxyethyl)anilin, Di-(2-methoxyethyl)amin

Neben der Härterkomponente a) bzw. a1) und a2) ist in der erfindungsgemäßen Mischung, dem erfindungsgemäßen Blend und dem erfindungsgemäßen Verfahren noch ein Härterkomponente b) der Formel I 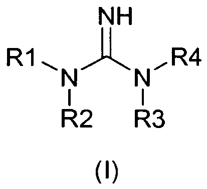 enthalten.

Die Reste R1 bis R3, R5 und R6 der Formel I in der Härterkomponente b) der erfindungsgemäßen Mischung, des erfindungsgemäßen Blends sowie des erfindungsgemäßen Verfahrens sind unabhängig voneinander ausgewählt aus der Gruppe von einem organischen Rest mit 1 bis 20 C-Atomen und Wasserstoff. Unter organischem Rest sind alle gesättigten, ungesättigten, cyclischen oder acyclischen Kohlenwasserstoffreste zu verstehen, die keine Heteroatome tragen. Besonders bevorzugt weist der organische Rest 1 bis10 C-Atome auf.
Unter organischem Rest, der ungesättigt und cyclisch ist, sind aromatische Gruppen zu nennen. Bevorzugte aromatische Kohlenwasserstoffreste sind ausgewählt aus der Gruppe von Phenyl-, Benzyl-, Xylen-, o-Tolyl-, eine durch eine oder mehrere C₂ bis C₄ Alkylgruppen substituierte Phenylgruppe und Benzylgruppe. Besonders bevorzugte aromatische Kohlenwasserstoffreste sind Phenylgruppen.
Die aliphatischen Kohlenwasserstoffreste sind ausgewählt aus der Gruppe der cyclischen und acyclischen Kohlenwasserstoffresten. Bevorzugt sind die acyclischen α-liphatischen Kohlenwasserstoffreste. Hierbei können als Kohlenwasserstoffreste bevorzugt solche mit C₁ bis C₁₀ Atome, besonders bevorzugt C₁ bis C₄ Atomen eingesetzt werden.
Ganz besonders bevorzugt sind die Reste für R1 bis R3, R5 und R6 ausgewählt aus der Gruppe von Methyl-, Ethyl-, n-Propyl-, Isopropyl-, n-Butyl-, sec-Butyl-, Phenyl- und o-Tolylreste. Insbesondere ganz besonders bevorzugt sind für die Reste R1 bis R3, R5 und R6 die aliphatischen Kohlenwasserstoffreste ausgewählt aus der Gruppe von Methyl-, Ethyl-, n-Propyl-, Isopropyl-, n-Butyl-, oder sec-Butylgruppe. Insbesondere ganz besonders bevorzugt sind Methyl-, Ethyl-, n-Propyl- und n-Butylgruppe.

R4 ist sowohl für die erfindungsgemäße Mischung, das erfindungsgemäße Blend als auch für das erfindungsgemäße Verfahren unabhängig von R1 bis R3, R5 und R6 ausgewählt aus der Gruppe von einem organischen Rest mit 1 bis 20 C-Atomen und einer -C(NH)NR5R6- Gruppe. Besonders bevorzugt ist R4 ausgewählt aus der Gruppe von Methyl-, Ethyl-, n-Propyl-, Isopropyl-, n-Butyl-, sec-Butyl-, Phenyl- und o-Tolylrest. Insbesondere ganz besonders bevorzugt sind Methyl-, Ethyl-, n-Propyl-, n-Butyl- und o-Tolylrest.

In einer besonders bevorzugten Ausführungsform sind R1 bis R4 unabhängig von einander organische aliphatische Kohlenwasserstoffe ausgewählt aus der Gruppe von Methyl-, Ethyl-, n-Propyl-, Isopropyl-, n-Butyl- und sec-Butylrest. Insbesondere ganz besonders bevorzugt sind Methyl-, Ethyl-, n-Propyl- und n-Butylgruppe.

Insbesondere ganz besonders bevorzugt handelt es sich bei der Verbindung von Formel I um Tetramethylguanidin.

Der Anteil der Verbindung der Formel I im erfindungsgemäßen Blend und im erfindungsgemäßen Verfahren liegt im Bereich von 0,5 bis 25 Gew.-%, bezogen auf die eingesetzte Menge des Epoxidharzes.

Der Anteil der Formel I in der erfindungsgemäßen Mischung liegt im Bereich von 5 bis 55 Gew.-%, bevorzugt im Bereich von 5 bis 30 Gew.-%, besonders bevorzugt zwischen 10 und 25 Gew.-%, bezogen auf die Menge der Mischung.

Bevorzugte erfindungsgemäße Mischungen und auch erfindungsgemäße Blends sind solche, die neben Tetramethylguanidin auch noch Polyetheramine ausgewählt aus der Gruppe von 3,6-dioxa-1,8-octandiamin, 4,7,10-Trioxa-1,13-tridecandiamin, 4,7-Dioxa-1,10-decandiamin,4,9-Dioxa-1,12-docecandiamin, difunktionellem, primären Polyetheramin auf Basis von Polypropylenglykol mit einer mittleren Molmasse von 2000, wie z.B. Jeffamine^{®} D-2000, Jeffamine^{®} XTJ-578 und Polyetheramine D 2000, difunktionellem, primären Polyetheramin auf der Basis von Polypropylenglykol mit einer mittleren Molmasse von 230, wie z.B. Jeffamine^{®} D-230 und Polyetheramine D 230, difunktionellem, primären Polyetheramin auf der Basis von Polypropylenglykol mit einer mittleren Molmasse von 400, wie z.B. Jeffamine^{®} D-400, Jeffamine^{®} XTJ-582 und Polyetheramine D 400, difunktionellem, primären Polyetheramin auf der Basis von Propylenoxid mit einer mittleren Molmasse von 4000, wie z.B. Jeffamine^{®} D-4000, difunktionellem, primären Polyetheramin hergestellt durch Aminierung eines mit Propylenoxid aufgepfropften Polyethylenglykols mit einer mittleren Molmasse von 2003, wie z.B. Jeffamine^{®} ED-2003, aliphatischem Polyetheramin auf der Basis von mit Propylenoxid gepfropftem Polyethylengylkol mit einer mittleren Molmasse von 900 wie z.B. Jeffamine^{®} ED-900, aliphatischem Polyetheramin auf Basis von mit Propylenoxid gepfropftem Polyethylenglykol mit einer mittleren Molmasse von 2000 wie z.B. Jeffamine^{®} ED- 2003, aliphatischem Polyetheramin auf der Basis von mit Propylenoxid gepfropftem Polyethylenglykol mit einer mittleren Molmasse von 600, wie z.B. Jeffamine^{®} ED-600 und dem Jeffamine^{®} XTJ 501, difunktionellem, primären Polyetheramin hergestellt durch Aminierung eines mit Propylenoxid aufgepfropften Diethylenglykols mit einer mittleren Molmasse von 220 wie z.B. Jeffamine^{®} HK-511, trifunktionellem, primären Polyetheramin hergestellt durch Reaktion von Propylenoxid mit Trimethylolpropan gefolgt durch eine Aminierung der endständigen OH-Gruppen mit einer mittleren Molmasse von 403, wie z.B. Jeffamine^{®} T-403 und Polyetheramine T 403, trifunktionellem, primären Polyetheramin hergestellt durch Reaktion von Propylenoxid mit Glycerin gefolgt durch eine Aminierung der endständigen OH-Gruppen mit einer mittleren Molmasse von 5000, wie z.B. Jeffamine^{®} T-5000 und Polyetheramine T 5000, aliphatischem Polyetheramin auf der Basis eines Copolymers aus Poly(tetramethylenetherglycol) und Polypropylenglycol mit einer mittleren Molmasse von 1000, wie z.B. Jeffamine^{®} XTJ-542, aliphatischem Polyetheramin auf der Basis eines Copolymers aus Poly(tetramethylenetherglycol) und Polypropylenglycol mit einer mittleren Molmasse von 1900, wie z.B. Jeffamine^{®} XTJ-548, aliphatischem Polyetheramin auf der Basis eines Copolymers aus Poly(tetramethylenetherglycol) und Polypropylenglycol mit einer mittleren Molmasse von 1400, wie z.B. Jeffamine^{®} XTJ-559, aliphatischem Polyethertriamin auf der Basis eines mit Butylenoxid gepfropftem mindestens dreiwertigen Alkohols mit einer mittleren Molmasse von 400, wie z.B. Jeffamine^{®} XTJ-566, aliphatischem Polyetheramin hergestellt durch Aminierung von mit Butylenoxid aufgepfropftem Alkoholen mit einer mittleren Molmasse von 219 wie z.B. Jeffamine^{®} XTJ-568, Polyetheramin auf der Basis von Pentaerythrit und Propylenoxid mit einer mittleren Molmasse von 600, wie z.B. Jeffamine^{®} XTJ-616, Polyetheramin auf Basis von Triethylenglykol mit einer mittleren Molmasse von 148, wie z.B. Jeffamine^{®} EDR 148, difunktionellem, primären Polyetheramin hergestellt durch Aminierung eines mit Propylenoxid aufgepfropftem Ethylenglykol mit einer mittleren Molmasse von 176, wie z.B. Jeffamine^{®} EDR 176 und einem Polyetheramin mit einer mittleren Molmasse von 400 hergestellt durch Aminierung von PolyTHF, das eine mittlere Molmasse von 250 aufweist wie z.B. PolyTHF Amin 350.

Besonders bevorzugte erfindungsgemäße Mischungen und auch erfindungsgemäße Blends sind zum einen solche, die neben Tetramethylguanidin und Polyetheraminen ausgewählt aus der Gruppe von difunktionellem, primären Polyetheramin auf der Basis von Polypropylenglykol mit einer mittleren Molmasse von 230, wie z.B. Jeffamine^{®} D-230 und Polyetheramine D 230, difunktionellem, primären Polyetheramin auf der Basis von Polypropylenglykol mit einer mittleren Molmasse von 400, wie z.B. Jeffamine^{®} D-400, Jeffamine® XTJ-582 und Polyetheramine D 400, difunktionellem, primären Polyetheramin hergestellt durch Aminierung eines mit Propylenoxid aufgepfropftem Diethylenglykols mit einer mittleren Molmasse von 220 wie z.B. Jeffamine^{®} HK-511, trifunktionellem, primären Polyetheramin hergestellt durch Reaktion von Propylenoxid mit Trimethylolpropan gefolgt durch eine Aminierung der endständigen OH-Gruppen mit einer mittleren Molmasse von 403, wie z.B. Jeffamine^{®} T-403 und Polyetheramine T 403, aliphatischem Polyetheramin auf der Basis von mit Propylenoxid gepfropftem Polyethylengylkol mit einer mittleren Molmasse von 900 wie z.B. Jeffamine^{®} ED-900, aliphatischem Polyetheramin auf der Basis eines Copolymers aus Poly(tetra-methylenetherglycol) und Polypropylenglycol mit einer mittleren Molmasse von 1000, wie z.B. Jeffamine^{®} XTJ-542, Polyetheramin auf der Basis eines Copolymers aus Poly(tetramethylenetherglycol) und Polypropylenglycol mit einer mittleren Molmasse von 1900, wie z.B. Jeffamine^{®} XTJ-548, aliphatischem Polyetheramin auf der Basis eines Copolymers aus Poly(tetramethylenetherglycol) und Polypropylenglycol mit einer mittleren Molmasse von 1400, wie z.B. Jeffamine^{®} XTJ-559, aliphatischem Polyethertriamin auf der Basis eines mit Butylenoxid gepfropftem mindestens dreiwertigen Alkohols mit einer mittleren Molmasse von 400, wie z.B. Jeffamine^{®} XTJ-566, aliphatischem Polyetheramin hergestellt durch Aminierung von mit Butylenoxid aufgepfropften Alkoholen mit einer mittleren Molmasse von 219 wie z.B. Jeffamine^{®} XTJ-568, auch noch ein Diamin ausgewählt aus der Gruppe von Isophorondiamin, 1,2-Diaminocyclohexan, 1-Methyl-2,4-diaminocyclohexan und 1,3-Bis(aminomethyl)cyclohexan enthalten. Eine ganz besonders bevorzugte erfindungsgemäße Mischung ist die, die Tetramethylguanidin, difunktionelles primäres Polyetheramin auf der Basis von Polypropylenglykol mit einer mittleren Molmasse von 230, wie z.B. Jeffamine® D-230 und Polyetheramine D 230 und Isophorondiamin enthalten.

Bei einer erfindungsgemäßen Mischung und einem bevorzugtem erfindungsgemäßen Blend, bei der neben der Verbindung der Formel I ein Polyetheramin und ein weiteres Amin mit einer Funktionalität ≥ 2 eingesetzt wird, ist das Polyetheramin im Verhältnis zum weiteren Amin im Bereich von 0,1 bis 10 zu 1 , bevorzugt im Bereich von 1,5 bis 10 zu 1, besonders bevorzugt im Bereich von 2,0 bis 5,0 zu 1. In einer ganz besonders bevorzugten erfindungsgemäßen Mischung und einem insbesondere ganz besonders bevorzugtem Blend, in der Tetramethylguanidin, Polyetheramin D230 / Jeffamine^{®} D230 und Isophorondiamin enthalten sind, ist das bevorzugte Mischungsverhältnis von Polyetheramin D230 / Jeffamine^{®} D230 zu Isophorondiamin im Bereich von 2,2 bis 2,6 zu 1, besonders bevorzugt im Bereich von 2,3 bis 2,5 zu 1.

Die erfindungsgemäße Mischung wird durch die dem Fachmann bekannten mechanischen Methoden aus den Einzelbestandteilen bei Temperaturen unterhalb von 160°C, bevorzugt im Bereich von 5 bis 30 °C gemischt.

Bei Benutzung der erfindungsgemäßen Mischung zum Härten von Epoxidharzen ist die Geschwindigkeit der Härtung vergleichbar oder besser gegenüber Härtungssystemen aus dem Stand der Technik.

Neben der Verwendung der erfindungsgemäßen Mischung in der Infusionstechnologien wie z.B. resin infusion, resin transfer molding (RTM), vacuum assisted resin transfer molding (VARTM), die in US 3,379,591 beschrieben sind, sind die erfindungsgemäßen Mischungen und erfindungsgemäßen Blends auch für weitere Technologien zum Aushärten von Epoxidharzen einsetzbar, die eine ausreichende Verarbeitungszeit bei Temperaturen von 15-45°C, kombiniert mit einer schnellen Aushärtung bei höheren Temperaturen, erfordern. Diese Technologien sind ausgewählt aus der Gruppe von Wickeln, Pultrusion, Hand-Lay-Up und Prepreg, wie sie in US 3,379,591 und
US 5,470,517 beschrieben sind. Beim Hand-Lay-Up-Verfahren wird ein Fasermaterial manuell oder maschinell mit Epoxidharz benetzt und dann werden diese Matten in eine Form gelegt und, im Falle dass mehrere Lagen verwendet werden, mit Walzen oder ähnlichen Geräten konsolidiert. Die Härtung erfolgt oft in einem Vakuumsack, da dadurch das Material konsolidiert wird und ein genauer Epoxidharzgehalt eingestellt werden kann.

Ein weiterer Gegenstand der vorliegenden Erfindung ist das gehärtete Epoxidharz, das erhältlich ist durch Aushärtung des erfindungsgemäßen Blends oder durch Härtung eines Epoxidharzes oder Epoxidharzgemisches mit der erfindungsgemäßen Mischung. Hierfür werden die erfindungsgemäßen Blends entweder in spezielle Formen eingefüllt oder auf Oberflächen aufgetragen und durch Temperaturerhöhung zur Aushärtung gebracht. In den Blends für eine Auftragung auf Oberflächen können noch weitere Füllstoffe im Blends enthalten sein. Diese Füllstoffe sind ausgewählt aus der Gruppe von Thixotropiermittel (hydrophile und hydrophobe pyrogene Kieselsäuren), UV-Stabilisatoren (nanoskalige Oxide wie Titandioxid und Zinkoxid), Flammschutzmittel (Polyphosphate und Phosphor), Silicate und Carbonate zur Verbesserung der mechanischen Eigenschaften. Die eingesetzten Formen, in die die erfindungsgemäßen Blends eingeführt werden, können faserverstärkendes Material oder aber Elemente enthalten, die vor Umwelteinflüssen wie Nässe, Sauerstoff, Staubkörner oder andere aggressiven Materialien oder Einflüsse geschützt werden müssen.

Bevorzugte gehärtete Epoxidharze sind solche, die in einem Formteil ausgehärtet sind. Diese Formteile sind ausgewählt aus der Gruppe von Formteilen für Kraftfahrzeuge, Flugzeuge, Schiffe, Boote, Sportartikel und Flügeln für Windkraftanlagen. Besonders bevorzugt sind Formteilen für Rotorblätter von Windkraftanlagen.

Diese Formteile können sowohl mit als auch ohne ein faserverstärkendes Material ausgelegt sein und/oder aber dem erfindungsgemäßen Blend und/oder der erfindungsgemäßen Mischung kann man noch faserverstärkende Materialien zugeben. Die Faserverstärkenden Materialien können somit Gewebe, Uni- und Multiaxiale Gelege, Vliese und Kurzfasern aus folgenden Fasermaterialien sein: Glasfasern, Kohlefasern, Aramidfasern, PE-Fasern (Dyneema) und Basaltfasern. Bevorzugt sind Gewebe und Uni- und Multiaxialgelege aus Glasfasern und Kohlefasern. Bei großen ,Bauteilen, die faserverstärkt sind, sind die Bauteile bevorzugt mit dem faserverstärkendem Material ausgelegt. Besonders bevorzugt sind Uni- und Multiaxialgelege aus Glasfasern. Die Flügelschalen für Windkraftanlagen werden bevorzugt mit Glasfaser-Gelegen ausgelegt.

Die Herstellung der Formkörpern erfolgt bevorzugt nach dem erfindungsgemäßen Verfahren bei dem eine entsprechende Form bereit gestellt wird, in diese Form das erfindungsgemäße Blend eingebracht wird und erst nach vollständiger Ausfüllung der Form komplett gehärtet wird. Bei dem erfindungsgemäßen Verfahren wird das erfindungsgemäße Blend, das die erfindungsgemäße Mischung enthalten kann bevorzugt über die Infusionstechnologie in die entsprechende Form eingebracht. Hierbei wird an das Formteil Vakuum angelegt. Dieses Vakuum saugt das Blend enthaltend Epoxidharz und gegebenenfalls die erfindungsgemäße Mischung bei Temperaturen unterhalb der Anfangshärtungstemperatur in die Form ein, so dass die Viskosität während des Füllvorgangs nahezu unverändert bleibt und alle Bereiche des Formteils vom Blend ausgefüllt werden, bevor dieses vollständig ausgehärtet wird. Anschließend erfolgt die vollständige Aushärtung des Blends im Formkörper. Zum vollständigen Aushärten können von außen weitere Heizquellen angelegt werden.

Die erfindungsgemäße Mischung kann in Gegenwart von Epoxidharzen auch als Strukturkleber von Composite Bauteilen miteinander und auch mit anderen Werkstoffen wie Metallen und Beton verwendet werden. Hierbei kann die erfindungsgemäße Mischung bzw. das erfindungsgemäße Blend mit faserartigen wie Glaskurzfasern und Füllstoffen wie Thixotropiermittel (hydrophile und hydrophobe pyrogene Kieselsäuren), UV - Stabilisatoren (nanoskalige Oxide wie Titandioxid und Zinkoxid), Flammschutzmittel (Polyphosphate und Phosphor), Silicate und Carbonate kombiniert werden. Gegenüber dem Stand der Technik kombinieren die Strukturkleber eine lange Verarbeitungszeit mit kurzen Härtungszeiten unter den oben genannten Härtungsbedingungen.

### Beispiele:

Als Stand der Technik wurde eine Mischung aus Polyetheramin D230 und Isophorondiamin im Gewichtsverhältnis 70/30 gewählt.

Das Blend, in dem eine Mischung aus Polyetheramin D230 und Isophorondiamin und Tetramethylguanidin eingesetzt wird, enthält 82 Gew. % handelsübliches Bisphenol-A-bisglycidylether (Epilox A19-03) und 18 Gew. % Butandiol-bisglycidylether (Epilox P13-21).

Die erfindungsgemäßen Mischungen zur Härtung des Epoxidharzsystems bestehen aus Mischungen von Polyetheramin D230 und Isophorondiamin (IPDA) im konstanten Gewichtsverhältnis 70/30, denen in unterschiedlichen Mengen an Tetramethylguanidin (TMG) beigemischt werden. Die Übersicht der getesteten Kombinationen findet sich in Tabelle 1.

**Tabelle 1: Zusammensetzung der untersuchten Kombinationen**

| Versuchs-Nr. | Einwaage | | | |
|---|---|---|---|---|
| Zeile/Spalte | Epoxidharzsystem | Polyetheramine D 230 | IPDA | TMG |
| 1/1 | 38,71 g | 7,90 g | 3,39 g | 0,00 g |
| 1/2 | 38,26 g | 7,81 g | 3,35 g | 0,59 g |
| 1/3 | 37,77 g | 7,71 g | 3,30 g | 1,22 g |
| 1/4 | 37,23 g | 7,60 g | 3,26 g | 1,92 g |
| 1/5 | 36,65 g | 7,48 g | 3,20 g | 2,67 g |
| 1/6 | 35,30 g | 7,20 g | 3,09 g | 4,41 g |
| 2/2 | 39,18 g | 7,20 g | 3,08 g | 0,54 g |
| 2/3 | 38,71 g | 7,11 g | 3,05 g | 1,13 g |
| 2/4 | 38,21 g | 7,02 g | 3,01 g | 1,77 g |
| 2/5 | 37,65 g | 6,91 g | 2,96 g | 2,47 g |
| 2/6 | 36,37 g | 6,68 g | 2,86 g | 4,09 g |
| 3/2 | 40,15 g | 6, 55 g | 2,81 g | 0,49 g |
| 3/3 | 39,71 g | 6,48 g | 2,78 g | 1,03 g |
| 3/4 | 39,24 g | 6,41 g | 2,75 g | 1,61 g |
| 3/5 | 38,71 g | 6,32 g | 2,71 g | 2,26 g |
| 3/6 | 37,50 g | 6,12 g | 2,62 g | 3,75 g |
| 4/2 | 41,16 g | 5,88 g | 2,52 g | 0,44 g |
| 4/3 | 40,76 g | 5,82 g | 2,50 g | 0,92 g |
| 4/4 | 40,32 g | 5,76 g | 2,47 g | 1,45 g |
| 4/5 | 39,84 g | 5,69 g | 2,44 g | 2,03 g |
| 4/6 | 38,71 g | 5,53 g | 2,37 g | 3,39 g |
| 5/2 | 42,23 g | 5,17 g | 2,22 g | 0,39 g |
| 5/3 | 41,86 g | 5,13 g | 2,20 g | 0,81 g |
| 5/4 | 41,47 g | 5,08 g | 2,18 g | 1,28 g |
| 5/5 | 41,03 g | 5,02 g | 2,15 g | 1,79 g |
| 5/6 | 40,00 g | 4,90 g | 2,10 g | 3,00 g |
| 6/2 | 43,35 g | 4,42 g | 1,90 g | 0,33 g |
| 6/3 | 43,03 g | 4,39 g | 1,88 g | 0,70 g |
| 6/4 | 42,68 g | 4,35 g | 1,87 g | 1,10 g |
| 6/5 | 42,29 g | 4,32 g | 1,85 g | 1,54 g |
| 6/6 | 41,38 g | 4,22 g | 1,81 g | 2,59 g |
| 7/2 | 44,53 g | 3,64 g | 1,56 g | 0,27 g |
| 7/3 | 44,26 g | 3,61 g | 1,55 g | 0,57 g |
| 7/4 | 43,97 g | 3,59 g | 1,54 g | 0,90 g |
| 7/5 | 43,64 g | 3,56 g | 1,53 g | 1,27 g |
| 7/6 | 42,86 g | 3,50 g | 1,50 g | 2,14 g |
| 8/2 | 45,79 g | 2,80 g | 1,20 g | 0,21 g |
| 8/3 | 45,57 g | 2,79 g | 1,20 g | 0,44 g |
| 8/4 | 45,34 g | 2,78 g | 1,19 g | 0,70 g |
| 8/5 | 45,07 g | 2,76 g | 1,18 g | 0,99 g |
| 8/6 | 44,45 g | 2,72 g | 1,17 g | 1,67 g |
| 9/2 | 47,11 g | 1,92 g | 0,82 g | 0,14 g |
| 9/3 | 46,96 g | 1,92 g | 0,82 g | 0,30 g |
| 9/4 | 46,79 g | 1,91 g | 0,82 g | 0,48 g |
| 9/5 | 46,60 g | 1,90 g | 0,82 g | 0,68 g |
| 9/6 | 46,16 g | 1,88 g | 0,81 g | 1,15 g |
| 10/2 | 48,51 g | 0,99 g | 0,42 g | 0,07 g |
| 10/3 | 48,43 g | 0,99 g | 0,42 g | 0,16 g |
| 10/4 | 48,34 g | 0,99 g | 0,42 g | 0,25 g |
| 10/5 | 48,24 g | 0,98 g | 0,42 g | 0,35 g |
| 10/6 | 48,00 g | 0,98 g | 0,42 g | 0,60 g |

In der folgenden Tabelle sind die Ergebnisse der Topfzeitbestimmung nach Methode B bei 60°C dargestellt.

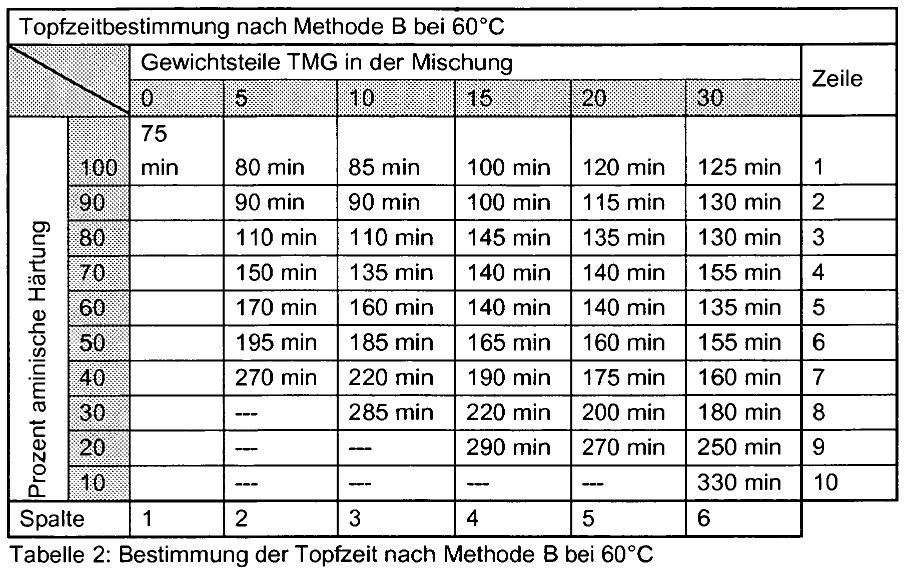

Für den Stand der Technik wurde eine Topfzeit (ToB) von 75 min ermittelt.
Die Versuche zeigen (Zeile 1), dass der Zusatz von TMG zu einer Mischung aus Polyetheraminen D230 und IPDA und die Verwendung dieser erfindungsgemäßen Blends zu einer Erhöhung der Topfzeit führt. Der Anteil von 30 Gew% TMG in der erfindungsgemäßen Mischung des erfindungsgemäßen Blends kann die Topfzeit um etwa 66% erhöhen.
Des Weiteren zeigen die Versuche (Spalte 3), dass eine Reduzierung des Anteil des Polyetheramins D230 und des IPDA (Prozent aminische Härtung) im Bereich von 0,3 bis 0,9 Aminäquivalent pro Äquivalent Epoxid eine deutlichere Erhöhnung der Topfzeit ergeben, als im Falle der Zugabe von TMG zu einer stöchiometrischen Mischung aus Polyetheramin D230 und IPDA. So wurde für die Kombination 30% aus Polyetheramin D230 und IPDA (aminische Härtung) und 10 Gew% TMG in der erfindungsgemäßen Mischung eine Erhöhung der Topfzeit um 380% erreicht.

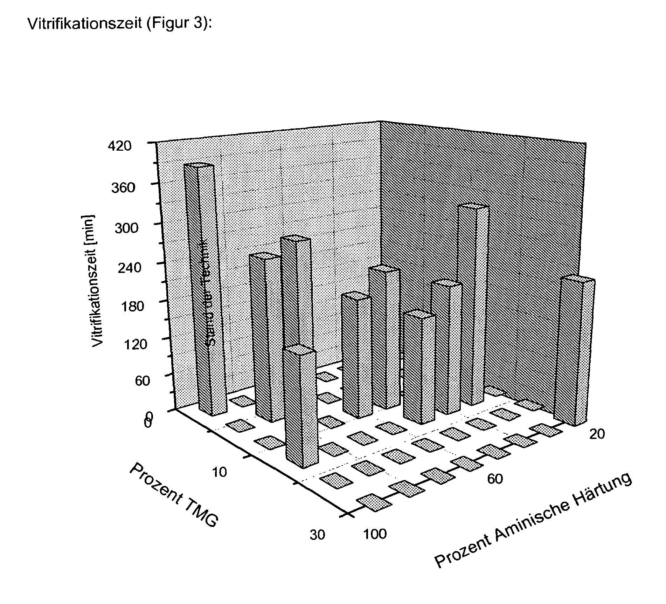

Zusätzlich zeigen die Versuche, dass die Geschwindigkeit der Härtung zwischen erfindungsgemäßen Beispielen und dem Vergleichsbeispiel vergleichbar oder besser ist. Dieser Effekt kann durch Bestimmung der Vitrifikationszeit für einige ausgewählte Systeme (1/1; 2/4; 3/2; 4/2; 5/3; 6/3; 6/4; 7/4; 8/4; 9/6) nachgewiesen werden (Figur 3). Die Vitrifikationszeit wurde mittels MDSC als Halbstufenhöhe des stufenförmigen Übergangs der spezifischen Wärmekapazität ermittelt. Diese Methode wird im Artikel: " Understanding vitrification during cure of epoxy resins using dynamic scanning calorimetry and rheological techniques." (Polymer, 41 (2000) 5949 ff) beschrieben.
Die erfindungsgemäße Mischung verringert die Vitrifikationszeit von > 6 Stunden auf eine Zeit ≤ 3 Stunden für eine Härtungstemperatur von 70°C im Vergleich zum Stand der Technik.

Da die Zusammensetzung der Mischung nicht nur die Reaktivität beeinflusst, sondern auch sich auf andere Parameter wie Glasübergangstemperatur und mechanische Kennwerte auswirkt wurden entsprechende Untersuchungen für die in Tabelle 1 genannten Systeme durchgeführt.

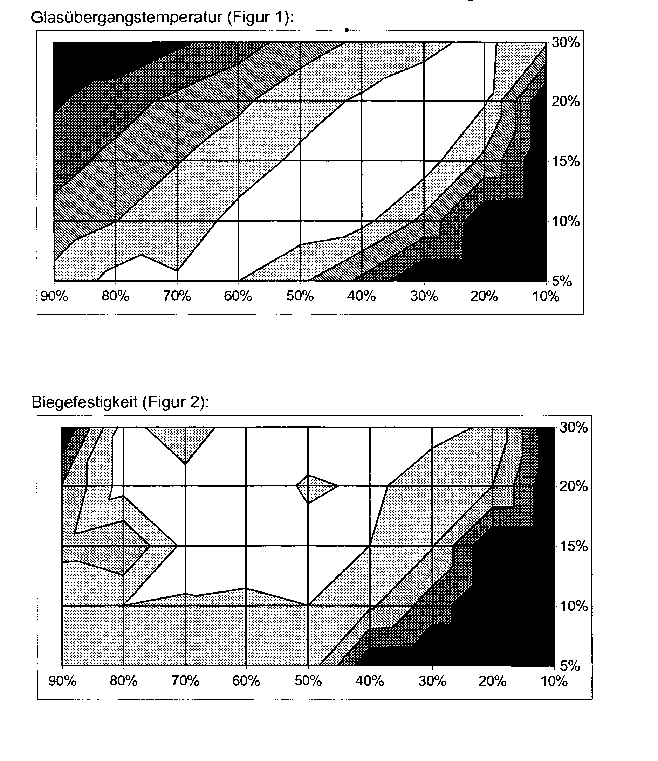

Dargestellt ist die Glasübergangstemperatur (Figur 1) als Funktion der Komposition. Auf der X-Achsen ist der Anteil der Summe von Polyetheramin D230 und IPDA (aminische Härtung) (entspricht Zeilen) und auf der γ-Achsen der Gewichtsanteil des TMG's (Härterkomponente b)) (entspricht Spalten) dargestellt. Die Farbe ändert sich abhängig von der erreichten Glasübergangstemperatur. Weiß bedeutet hohe und schwarz bedeutet niedrige Glasübergangstemperatur.

Dargestellt ist die Biegefestigkeit (Figur 2) als Funktion der Komposition. Auf der X-Achsen ist der Anteil der der Summe von Polyetheramin D230 und IPDA (aminische Härtung) (entspricht Zeilen) und auf der y-Achsen der Gewichtsanteil des TMG's (Härterkomponente b)) (entspricht Spalten) dargestellt. Die Farbe ändert sich abhängig von der erreichten Biegefestigkeit. Weiß bedeutet hohe und schwarz bedeutet niedrige Biegefestigkeit.

Betrachtet man die all diese Ergebnisse, so ergibt sich, dass die erfindungsgemäße Mischung eine optimale Kombination aller Parameter: Verarbeitung, Härtungsdauer und mechanischer bzw. thermischer Eigenschaften darstellt.

## Patentansprüche

1. Blend enthaltend
α) ein oder mehrere Epoxidharze und
β) eine Mischung enthaltend
1) 0,3 bis 0,9 Aminäquivalente, pro Äquivalent Epoxid der eingesetzten Komponente α), an einer Härterkomponente a) und
2) eine Härterkomponente b),
**dadurch gekennzeichnet, dass** die Härterkomponente a) ein oder mehrere Amine mit einer Funktionalität ≥ 2 enthält und mindestens ein Amin beim stöchiometrischen Vermischen mit dem Epoxidharz im 100 g Ansatz bei Raumtemperatur zu einer Aushärtungszeit von weniger als 24 h führt und die Härterkomponente b) mindestens eine Verbindung der Formel I 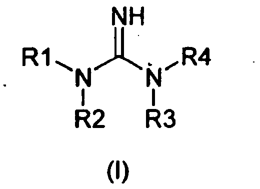 enthält, wobei der Anteil der Härterkomponente b) 5 bis 55 Gew.-% bezogen auf den Gewichtsanteil der Mischung beträgt und in Formel I R1 bis R3, R5 und R6 unabhängig voneinander einen organischen Rest mit 1 bis 20 C-Atomen und Wasserstoff bedeutet und R4 ausgewählt ist aus der Gruppe von einem organischen Rest mit 1 bis 20 C-Atomen und einer -C(NH)NR5R6 Gruppe.

2. Blend nach Anspruch 1, **dadurch gekennzeichnet, dass** die Härterkomponente a) ausgewählt ist aus der Gruppe von Aminen mit einer Funktionalität 2 2.

3. Blend nach einem der Ansprüche 1 bis 2, **dadurch gekennzeichnet, dass**, die Härterkomponente a) mindestens zwei Härterkomponenten a1) und a2) enthält, wobei die Härterkomponente a1) mindestens ein Polyetheramin mit einer Funktionalität ≥ 2 ist und die Härterkomponente a2) mindestens ein weiteres Amin mit einer Funktionalität ≥ 2 ist.

4. Blend nach einem der Ansprüche 1 bis 3, **dadurch gekennzeichnet, dass**
die Härterkomponente a1) ausgewählt ist aus der Gruppe von 3,6-dioxa-1,8-octandiamin, 4,7,10-Trioxa-1,13-tridecandiamin, 4,7-Dioxa-1,10-decandiamin, 4,9-Dioxa-1,12-docecandiamin, Polyetheramin auf der Basis von Triethylen-glykol mit einer mittleren Molmasse von 148, difunktionellem, primären Polyetheramin hergestellt durch Aminierung eines mit Propylenoxid aufgepfropften Ethylenglykols mit einer mittleren Molmasse von 176, difunktionellem, primären Polyetheramin auf der Basis von Propylenoxid mit einer mittleren Molmasse von 4000, difunktionellem, primären Polyetheramin hergestellt durch Aminierung eines mit Propylenoxid aufgepfropften Polyethylenglykols mit einer mittleren Molmasse von 2003, aliphatischem Polyetheramin auf der Basis von mit Propylenoxid gepfropftem Polyethylengylkol mit einer mittleren Molmasse von 900, aliphatischem Polyetheramin auf der Basis von mit Propylenoxid gepfropftem Polyethylenglykol mit einer mittleren Molmasse von 600, difunktionellem, primären Polyetheramin hergestellt durch Aminierung eines mit Propylenoxid aufgepfropften Diethylenglykols mit einer mittleren Molmasse von 220, aliphatischem Polyetheramin auf der Basis eines Copolymers aus Poly(tetramethylenetherglycol) und Polypropylenglycol mit einer mittleren Molmasse von 1000, aliphatischem Polyetheramin auf der Basis eines Copolymers aus Poly(tetramethylenetherglycol) und Polypropylenglycol mit einer mittleren Molmasse von 1900, aliphatischem Polyetheramin auf der Basis eines Copolymers aus Poly(tetramethylenetherglycol) und Polypropylenglycol mit einer mittleren Molmasse von 1400, Polyethertriamin auf der Basis eines mit Butylenoxid gepfropftem mindestens dreiwertigen Alkohols mit einer mittleren Molmasse von 400, aliphatischem Polyetheramin hergestellt durch Aminierung von mit Butylenoxid aufgepfropften Alkoholen mit einer mittleren Molmasse von 219, Polyetheramin auf der Basis von Pentaerythrit und Propylenoxid mit einer mittleren Molmasse von 600, difunktionellem, primären Polyetheramin auf Basis von Polypropylenglykol mit einer mittleren Molmasse von 2000, difunktionellem, primären Polyetheramin auf der Basis von Polypropylenglykol mit einer mittleren Molmasse von 230, difunktionellem, primären Polyetheramin auf der Basis von Polypropylenglykol mit einer mittleren Molmasse von 400, trifunktionellem, primären Polyetheramin hergestellt durch Reaktion von Propylenoxid mit Trimethylolpropan gefolgt durch eine Aminierung der endständigen OH-Gruppen mit einer mittleren Molmasse von 403, trifunktionellem, primären Polyetheramin hergestellt durch Reaktion von Propylenoxid mit Glycerin gefolgt durch eine Aminierung der endständigen OH-Gruppen mit einer mittleren Molmasse von 5000 und einem Polyetheramin mit einer mittleren Molmasse von 400 hergestellt durch Aminierung von PolyTHF, das eine mittlere Molmasse von 250 aufweist und
die Härterkomponente a2) ausgewählt aus der Gruppe von 1,12-Diaminododecan, 1,10-Diaminodecan, 1,2-Diaminocyclohexan, 1,2-Propandiamin, 1,3-Bis(aminomethyl)cyclohexan, 1,3-Propandiamin, 1- Methyl-2,4-diaminocyclohexan, 2,2'-Oxybis(ethylamin), 3,3'-Dimethyl-4,4'-diaminodicyclohexylmethan, 4,4'-Methylendianilin, 4-Ethyl-4-methylamino-1-octylamin, Diethylentriamin, Ethylendiamin, Hexamethylendiamin, Isophorondiamin, Menthendiamin, Xylylendiamin, N-Aminoethylpiperazin, Neopentandiamin, Norbomandiamin, Octanmethylendiamin, Piperazin, 4,8-Diamino-tricyclo[5.2.1.0]decan, Tolylendiamin, Triethylentetramin und Trimethylhexamethylendiamin ist.

5. Blend nach einem der Ansprüche 1 bis 4, **dadurch gekennzeichnet, dass** die Reste R1 bis R3, R5 und R6 der Verbindungen der Formel I unabhängig voneinander ausgewählt sind aus der Gruppe von Wasserstoff, Methyl, Ethyl, n-Propyl, i-Propyl, n-Butyl, sec.- Butyl, Phenyl und o-Tolyl und R4 ausgewählt ist aus der Gruppe von Methyl, Ethyl, n-Propyl, i-Propyl, n-Butyl, sec.- Butyl, Phenyl, o-Tolyl und einer -C(NH)NR5R6- Gruppe.

6. Blend nach Anspruch 1, **dadurch gekennzeichnet, dass** die Mischung als Härterkomponente a1) ein Polyetheramin mit einer Funktionalität von ≥ 2 ausgewählt aus der Gruppe von difunktionellem, primären Polyetheramin auf der Basis von Polypropylenglykol mit einer mittleren Molmasse von 230, difunktionellem, primären Polyetheramin auf der Basis von Polypropylenglykol mit einer mittleren Molmasse von 400, aliphatischem, difunktionellem, primären Polyetheramin auf Basis von Polypropylenglykol mit einer mittleren Molmasse von 2000, difunktionellem, primären Polyetheramin hergestellt durch Aminierung eines mit Propylenoxid aufgepfropften Diethylenglykols mit einer mittleren Molmasse von 220, trifunktionellem, primären Polyetheramin hergestellt durch Reaktion von Propylenoxid mit Trimethylolpropan gefolgt durch eine Aminierung der endständigen OH-Gruppen mit einer mittleren Molmasse von 403, aliphatischem Polyetheramin auf der Basis von mit Propylenoxid gepfropftem Polyethylengylkol mit einer mittleren Molmasse von 900, aliphatischem Polyetheramin auf der Basis eines Copolymers aus Poly-(tetramethylenetherglycol) und Polypropylenglycol mit einer mittleren Molmasse von 1000, aliphatischem Polyetheramin auf der Basis eines Copolymers aus Poly(tetramethylenetherglycol) und Polypropylenglycol mit einer mittleren Molmasse von 1900, aliphatischem Polyetheramin auf der Basis eines Copolymers aus Poly(tetramethylenetherglycol) und Polypropylenglycol mit einer mittleren Molmasse von 1400, Polyethertriamin auf der Basis eines mit Butylenoxid gepfropftem mindestens dreiwertigen Alkohols mit einer mittleren Molmasse von 400, aliphatischem Polyetheramin hergestellt durch Aminierung von mit Butylenoxid aufgepfropftem Alkoholen mit einer mittleren Molmasse von 219, trifunktionellem, primäres Polyetheramin hergestellt durch Reaktion von Propylenoxid mit Glycerin gefolgt durch eine Aminierung der endständigen OH-Gruppen mit einer mittleren Molmasse von 5000 und als Härterkomponente a2) ein weiteres Amin mit einer Funktionalität ≥ 2 ausgewählt aus der Gruppe von Isophorondiamin, Aminoethylpiperazin, 1,3 Bis(aminoethyl)cyclohexan, Triethylentetramin, wobei das Verhältnis von a1) zu a2) im Bereich von 0,1 bis 10 zu 1 liegt

7. Blend nach einem der Ansprüche 1 bis 6, **dadurch gekennzeichnet, dass** die Mischung als Härterkomponente a1) ein Polyetheramin ausgewählt aus der Gruppe von Polyetheramin D 230, Polyetheramin D400, Polyetheramin T 403, Polyetheramin T 5000, die Härterkomponente a2) ausgewählt ist aus der Gruppe von Isophorondiamin, Aminoethylpiperazin, 1,3-Bis(aminomethyl)cyclohexan und Triethylentetraamin und die Härterkomponente b) Tetramethylguanidin ist und das Verhältnis von Härterkomponente a1) zu Härterkomponente a2) im Bereich von 1,5 bis 10 zu 1 liegt und 10 bis 60 Mol % weniger an Mischung zum Epoxidharz gegeben wird als zur Umsetzung der aktiven Epoxygruppen an Aminfunktionen der Mischung benötigt werden.

8. Blend nach einem der Ansprüche 1 bis 7, **dadurch gekennzeichnet, dass** das Blend noch faserverstärktes Material enthält.

9. Verfahren zur Herstellung des Blends nach einem der Ansprüche 1 bis 8, **dadurch gekennzeichnet, dass** ein oder mehrere Epoxidharze mit der Mischung bei Temperaturen unterhalb der Anfangshärtungstemperatur der Härterkomponente a) vermischt werden.

10. Verwendung des Blends nach einem der Ansprüche 1 bis 8 zur Herstellung von gehärteten Epoxidharzen.

11. Verwendung nach Anspruch 10, **dadurch gekennzeichnet, dass** die gehärteten Epoxidharze Formkörper sind.

12. Verwendung nach Anspruch 11, **dadurch gekennzeichnet, dass** die Formkörper verstärkendes Material enthalten.

13. Gehärtetes Epoxidharz erhältlich durch Härtung des Blends nach einem der Ansprüche 1 bis 8.

14. Gehärtetes Epoxidharz nach Anspruch 13, **dadurch gekennzeichnet, dass** das gehärtete Epoxidharz einen Formkörper darstellt.

15. Formkörper nach Anspruch 14, **dadurch gekennzeichnet dass** er faserverstärkt ist.

16. Formkörper nach Anspruch 15, erhältlich durch Aushärtung einer Form die mit einem faserverstärktem Material ausgelegt ist und Einbringung des Blends nach einem der Ansprüche 1 bis 7 mittels Infusionstechnologie in die Form.

17. Formkörper nach einem der Ansprüche 15 bis 16, **dadurch gekennzeichnet, dass** der Formkörper verstärkte Bauteile für Rotorblätter von Windkraftanlagen darstellt.

## Claims

1. A blend comprising
α) one or more epoxy resins and
β) a mixture comprising
1) 0.3 to 0.9 amine equivalent, per equivalent of epoxide of component □) used, of a hardener component a) and
2) a hardener component b),
wherein the hardener component a) comprises one or more amines having a functionality ≥ 2, and at least one amine, when mixed stoichiometrically with the epoxy resin in the 100 g batch, leads at room temperature to a cure time of less than 24 h, and the hardener component b) comprises at least one compound of the formula I 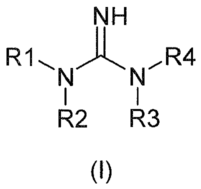 where the fraction of the hardener component b) is 5% to 55% by weight, based on the weight fraction of the mixture, and in formula I R1 to R3, R5 and R6 each independently are an organic radical having 1 to 20 C atoms and hydrogen, and R4 is selected from the group of an organic radical having 1 to 20 C atoms and a group -C(NH)NR5R6.

2. The blend according to claim 1, wherein the hardener component a) is selected from the group of amines having a functionality ≥ 2.

3. The blend according to either of claims 1 and 2, wherein the hardener component a) comprises at least two hardener components a1) and a2), the hardener component a1) being at least one polyetheramine having a functionality ≥ 2 and the hardener component a2) being at least one further amine having a functionality ≥ 2.

4. The blend according to any of claims 1 to 3,
wherein
the hardener component a1) is selected from the group of 3,6-dioxa-1,8-octanediamine, 4,7,10-trioxa-1,13-tridecanediamine, 4,7-dioxa-1,10-decanediamine, 4,9-dioxa-1,12-dodecanediamine, polyetheramine based on triethylene glycol with an average molar mass of 148, difunctional, primary polyetheramine prepared by aminating an ethylene glycol grafted with propylene oxide, with an average molar mass of 176, difunctional, primary polyetheramine based on propylene oxide with an average molar mass of 4000, difunctional, primary polyetheramine prepared by aminating a polyethylene glycol grafted with propylene oxide, with an average molar mass of 2003, aliphatic polyetheramine based on polyethylene glycol grafted with propylene oxide, with an average molar mass of 900, aliphatic polyetheramine based on polyethylene glycol grafted with propylene oxide, with an average molar mass of 600, difunctional, primary polyetheramine prepared by aminating a diethylene glycol grafted with propylene oxide, with an average molar mass of 220, aliphatic polyetheramine based on a copolymer of poly(tetramethylene ether glycol) and polypropylene glycol with an average molar mass of 1000, aliphatic polyetheramine based on a copolymer of poly(tetramethylene ether glycol) and polypropylene glycol with an average molar mass of 1900, aliphatic polyetheramine based on a copolymer of poly(tetramethylene ether glycol) and polypropylene glycol with an average molar mass of 1400, polyethertriamine based on an at least trihydric alcohol grafted with butylene oxide, with an average molar mass of 400, aliphatic polyetheramine prepared by aminating alcohols grafted with butylene oxide, with an average molar mass of 219, polyetheramine based on pentaerythritol and propylene oxide with an average molar mass of 600, difunctional, primary polyetheramine based on polypropylene glycol with an average molar mass of 2000, difunctional, primary polyetheramine based on polypropylene glycol with an average molar mass of 230, difunctional, primary polyetheramine based on polypropylene glycol with an average molar mass of 400, trifunctional, primary polyetheramine prepared by reacting propylene oxide with trimethylolpropane, followed by amination of the terminal OH groups, with an average molar mass of 403, trifunctional, primary polyetheramine prepared by reacting propylene oxide with glycerol, followed by amination of the terminal OH groups, with an average molar mass of 5000, and a polyetheramine having an average molar mass of 400, prepared by aminating polyTHF which has an average molar mass of 250, and
the hardener component a2) is selected from the group of 1,12-diaminododecane, 1,10-diaminodecane, 1,2-diaminocyclohexane, 1,2-propanediamine, 1,3-bis(aminomethyl)cyclohexane, 1,3-propanediamine, 1-methyl-2,4-diaminocyclohexane, 2,2'-oxybis(ethylamine), 3,3'-dimethyl-4,4'-diaminodicyclohexylmethane, 4,4'-methylenedianiline, 4-ethyl-4-methylamino-1-octylamine, diethylenetriamine, ethylenediamine, hexamethylenediamine, isophoronediamine, menthenediamine, xylylenediamine, N-aminoethylpiperazine, neopentanediamine, norbornanediamine, octamethylenediamine, piperazine, 4,8-diaminotri-cyclo[5.2.1.0]decane, tolylenediamine, triethylenetetramine, and trimethylhexamethylenediamine.

5. The blend according to any of claims 1 to 4, wherein the radicals R1 to R3, R5, and R6 of the compounds of the formula I are each independently selected from the group of hydrogen, methyl, ethyl, n-propyl, isopropyl, n-butyl, sec-butyl, phenyl, and o-tolyl, and R4 is selected from the group of methyl, ethyl, n-propyl, isopropyl, n-butyl, sec-butyl, phenyl, o-tolyl, and a group -C(NH)NR5R6-.

6. The blend according to claim 1, wherein the mixture comprises as hardener component a1) a polyetheramine having a functionality of ≥ 2, selected from the group of difunctional, primary polyetheramine based on polypropylene glycol, with an average molar mass of 230, difunctional, primary polyetheramine based on polypropylene glycol, with an average molar mass of 400, aliphatic, difunctional, primary polyetheramine based on polypropylene glycol, with an average molar mass of 2000, difunctional, primary polyetheramine prepared by aminating a diethylene glycol grafted with propylene oxide, with an average molar mass of 220, trifunctional, primary polyetheramine prepared by reacting propylene oxide with trimethylolpropane, followed by amination of the terminal OH groups, with an average molar mass of 403, aliphatic polyetheramine based on polyethylene glycol grafted with propylene oxide, with an average molar mass of 900, aliphatic polyetheramine based on a copolymer of poly(tetramethylene ether glycol) and polypropylene glycol, with an average molar mass of 1000, aliphatic polyetheramine based on a copolymer of poly(tetramethylene ether glycol) and polypropylene glycol, with an average molar mass of 1900, aliphatic polyetheramine based on a copolymer of poly(tetramethylene ether glycol) and polypropylene glycol, with an average molar mass of 1400, polyethertriamine based on an at least trihydric alcohol grafted with butylene oxide, with an average molar mass of 400, aliphatic polyetheramine prepared by aminating alcohols grafted with butylene oxide, with an average molar mass of 219, trifunctional, primary polyetheramine prepared by reacting propylene oxide with glycerol, followed by amination of the terminal OH groups, with an average molar mass of 5000, and as hardener component a2) a further amine having a functionality ≥ 2, selected from the group of isophoronediamine, aminoethylpiperazine, 1,3 bis(aminoethyl)cyclohexane, and triethylenetetramine, the ratio of a1) to a2) being in the range from 0.1 to 10:1.

7. The blend according to any of claims 1 to 6, wherein the mixture comprises as hardener component a1) a polyetheramine selected from the group of polyetheramine D 230, polyetheramine D 400, polyetheramine T 403, polyetheramine T 5000, the hardener component a2) is selected from the group of isophoronediamine, aminoethylpiperazine, 1,3-bis(aminomethyl)cyclohexane, and triethylenetetraamine, and the hardener component b) is tetramethylguanidine, and the ratio of hardener component a1) to hardener component a2) is in the range from 1.5 to 10:1, and 10 to 60 mol% less of mixture is added to the epoxy resin than is needed for the reaction of the active epoxy groups at amine functions of the mixture.

8. The blend according to any of claims 1 to 7, wherein the blend further comprises fiber-reinforced material.

9. A process for preparing the blend according to any of claims 1 to 8, which comprises mixing one or more epoxy resins with the mixture at temperatures below the initial curing temperature of the hardener component a).

10. The use of the blend according to any of claims 1 to 8 for producing a cured epoxy resin.

11. The use according to claim 10, wherein the cured epoxy resin is a molding.

12. The use according to claim 11, wherein the molding comprises reinforcing material.

13. A cured epoxy resin obtainable by curing the blend according to any of claims 1 to 8.

14. The cured epoxy resin according to claim 13, wherein the cured epoxy resin is a molding.

15. A molding according to claim 14, which is fiber-reinforced.

16. The molding according to claim 15, obtainable by curing a mold lined with a fiber-reinforced material and introducing the blend according to any of claims 1 to 7 into the mold by means of infusion technology.

17. The molding according to either of claims 15 and 16, wherein the molding is a reinforced component for rotor blades of wind turbines.

## Revendications

1. Alliage contenant
α) une ou plusieurs résines époxyde et
β) un mélange contenant
1) 0,3 à 0,9 équivalent d'amine, par équivalent d'époxyde du composant α) utilisé, d'un composant agent de durcissement a), et
2) un composant agent de durcissement b), **caractérisé en ce que** le composant agent de durcissement a) contient une ou plusieurs amines d'une fonctionnalité ≥ 2 et au moins une amine conduit à un temps de durcissement inférieur à 24 h lors du mélange stoechiométrique avec la résine époxyde dans la préparation de 100 g à température ambiante, et le composant agent de durcissement b) contient au moins un
composé de formule I 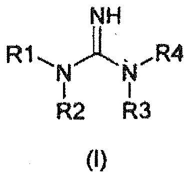 la proportion du composant agent de durcissement b) étant de 5 à 55 % en poids par rapport à la proportion en poids du mélange et, dans la formule I, R1 à R3, R5 et R6 signifiant indépendamment les uns des autres un radical organique de 1 à 20 atomes C et l'hydrogène, et R4 étant choisi dans le groupe constitué par un radical organique de 1 à 20 atomes C et un groupe -C(NH)NR5R6.

2. Alliage selon la revendication 1, **caractérisé en ce que** le composant agent de durcissement a) est choisi dans le groupe constitué par les amines d'une fonctionnalité ≥ 2.

3. Alliage selon l'une quelconque des revendications 1 à 2, **caractérisé en ce que** le composant agent de durcissement a) contient au moins deux composants agent de durcissement a1) et a2), le composant agent de durcissement a1) étant au moins une polyéther-amine d'une fonctionnalité ≥ 2 et le composant agent de durcissement a2) étant au moins une autre amine d'une fonctionnalité ≥ 2.

4. Alliage selon l'une quelconque des revendications 1 à 3, **caractérisé en ce que**
le composant agent de durcissement a1)
est choisi dans le groupe constitué par la 3,6-dioxa-1,8-octane-diamine, la 4,7,10-trioxa-1,13-tridécane-diamine, la 4,7-dioxa-1,10-décane-diamine, la 4,9-dioxa-1,12-dodécane-diamine, une polyéther-amine à base de triéthylène glycol d'une masse moléculaire moyenne de 148, une polyéther-amine primaire bifonctionnelle fabriquée par amination d'un éthylène glycol greffé avec de l'oxyde de propylène d'une masse moléculaire moyenne de 176, une polyéther-amine primaire bifonctionnelle à base d'oxyde de propylène d'une masse moléculaire moyenne de 4 000, une polyéther-amine primaire bifonctionnelle fabriquée par amination d'un polyéthylène glycol greffé avec de l'oxyde de propylène d'une masse moléculaire moyenne de 2 003, une polyéther-amine aliphatique à base de polyéthylène glycol greffé avec de l'oxyde de propylène d'une masse moléculaire moyenne de 900, une polyéther-amine aliphatique à base de polyéthylène glycol greffé avec de l'oxyde de propylène d'une masse moléculaire moyenne de 600, une polyéther-amine primaire bifonctionnelle fabriquée par amination d'un diéthylène glycol greffé avec de l'oxyde de propylène d'une masse moléculaire moyenne de 220, une polyéther-amine aliphatique à base d'un copolymère de poly(tétraméthylène éther glycol) et de polypropylène glycol d'une masse moléculaire moyenne de 1 000, une polyéther-amine aliphatique à base d'un copolymère de poly(tétraméthylène éther glycol) et de polypropylène glycol d'une masse moléculaire moyenne de 1 900, une polyéther-amine aliphatique à base d'un copolymère de poly(tétraméthylène éther glycol) et de polypropylène glycol d'une masse moléculaire moyenne de 1 400, une polyéther-triamine à base d'un alcool au moins trivalent greffé avec de l'oxyde de butylène d'une masse moléculaire moyenne de 400, une polyéther-amine aliphatique fabriquée par amination d'alcools greffés avec de l'oxyde de butylène d'une masse moléculaire moyenne de 219, une polyéther-amine à base de pentaérythrite et d'oxyde de propylène d'une masse moléculaire moyenne de 600, une polyéther-amine primaire bifonctionnelle à base de polypropylène glycol d'une masse moléculaire moyenne de 2 000, une polyéther-amine primaire bifonctionnelle à base de polypropylène glycol d'une masse moléculaire moyenne de 230, une polyéther-amine primaire bifonctionnelle à base de polypropylène glycol d'une masse moléculaire moyenne de 400, une polyéther-amine primaire trifonctionnelle fabriquée par une réaction d'oxyde de propylène avec du triméthylolpropane suivie par une amination des groupes OH terminaux d'une masse moléculaire moyenne de 403, une polyéther-amine primaire trifonctionnelle fabriquée par une réaction d'oxyde de propylène avec de la glycérine suivie par une amination des groupes OH terminaux d'une masse moléculaire moyenne de 5 000, et une polyéther-amine d'une masse moléculaire moyenne de 400 fabriquée par amination de polyTHF, qui présente une masse moléculaire moyenne de 250,
le composant agent de durcissement a2)
est choisi dans le groupe constitué par le 1,12-diaminododécane, le 1,10-diaminodécane, le 1,2-diaminocyclohexane, la 1,2-propane-diamine, le 1,3-bis(aminométhyl)cyclohexane, la 1,3-propane-diamine, le 1-méthyl-2,4-diaminocyclohexane, la 2,2°-oxybis(éthylamine), le 3,3'-diméthyl-4,4'-diamino-dicyclohexylméthane, la 4,4-éthylène-dianiline, la 4-éthyl-4-méthylamino-1-octylamine, la diéthylène-triamine, l'éthylène-diamine, l'hexaméthylène-diamine, l'isophorone-diamine, la menthène-diamine, la xylylène-diamine, la N-aminoéthylpipérazine, la néopentane-diamine, la norbornane-diamine, l'octaneméthylène-diamine, la pipérazine, le 4,8-diamino-tricyclo[5.2.1.0]décane, la tolylène-diamine, la triéthylène-tétramine, la triméthylhexaméthylène-diamine.

5. Alliage selon l'une quelconque des revendications 1 à 4, **caractérisé en ce que** les radicaux R1 à R3, R5 et R6 des composés de formule I sont choisis indépendamment les uns des autres dans le groupe constitué par l'hydrogène, méthyle, éthyle, n-propyle, i-propyle, n-butyle, sec.-butyle, phényle et o-tolyle, et R4 est choisi dans le groupe constitué par méthyle, éthyle, n-propyle, i-propyle, n-butyle, sec.-butyle, phényle, o-tolyle et un groupe -C(NH)NR5R6.

6. Alliage selon la revendication 1, **caractérisé en ce que** le mélange contient en tant que composant agent de durcissement a1) une polyéther-amine d'une fonctionnalité ≥ 2 choisie dans le groupe constitué par une polyéther-amine primaire bifonctionnelle à base de polypropylène glycol d'une masse moléculaire moyenne de 230, une polyéther-amine primaire bifonctionnelle à base de polypropylène glycol d'une masse moléculaire moyenne de 400, une polyéther-amine primaire bifonctionnelle aliphatique à base de polypropylène glycol d'une masse moléculaire moyenne de 2 000, une polyéther-amine primaire bifonctionnelle fabriquée par amination de diéthylène glycol greffé avec de l'oxyde de propylène d'une masse moléculaire moyenne de 220, une polyéther-amine primaire trifonctionnelle fabriquée par une réaction d'oxyde de propylène avec du triméthylolpropane suivie une amination des groupes OH terminaux d'une masse moléculaire moyenne de 403, une polyéther-amine aliphatique à base de polyéthylène glycol greffé avec de l'oxyde de propylène d'une masse moléculaire moyenne de 900, une polyéther-amine aliphatique à base d'un copolymère de poly(tétraméthylène éther glycol) et de polypropylène glycol d'une masse moléculaire moyenne de 1 000, une polyéther-amine aliphatique à base d'un copolymère de poly(tétraméthylène éther glycol) et de polypropylène glycol d'une masse moléculaire moyenne de 1 900, une polyéther-amine aliphatique à base d'un copolymère de poly(tétraméthylène éther glycol) et de polypropylène glycol d'une masse moléculaire moyenne de 1 400, une polyéther-triamine à base d'un alcool au moins trivalent greffé avec de l'oxyde de butylène d'une masse moléculaire de 400, une polyéther-amine aliphatique fabriquée par amination d'alcools greffés avec de l'oxyde de butylène d'une masse moléculaire moyenne de 219, une polyéther-amine primaire trifonctionnelle fabriquée par une réaction d'oxyde de propylène avec de la glycérine suivie par une amination des groupes OH terminaux d'une masse moléculaire moyenne de 5 000, et en tant que composant agent de durcissement a2) une autre amine d'une fonctionnalité > 2 choisie dans le groupe constitué par l'isophoronediamine, l'aminoéthylpipérazine, le 1,3-bis(aminoéthyl)cyclohexane, la triéthylène-tétramine, le rapport entre a1) et a2) étant dans la plage allant de 0,1 à 10 sur 1.

7. Alliage selon l'une quelconque des revendications 1 à 6, **caractérisé en ce que** le mélange contient en tant que composant agent de durcissement a1) une polyéther-amine choisie dans le groupe constitué par la polyéther-amine D 230, la polyéther-amine D 400, la polyéther-amine T 403, la polyéther-amine T 5000, le composant agent de durcissement a2) est choisi dans le groupe constitué par l'isophorone-diamine, l'aminoéthylpipérazine, le 1,3-bis(aminométhyl)cyclohexane et la triéthylène-tétraamine, et le composant agent de durcissement b) est la tétraméthylguanidine, et le rapport entre le composant agent de durcissement a1) et le composant agent de durcissement a2) est dans la plage allant de 1,5 à 10 sur 1, et 10 à 60 % en moles de moins de mélange que nécessaire pour la réaction des groupes époxy actifs sur les fonctions amine du mélange est ajouté à la résine époxyde.

8. Alliage selon l'une quelconque des revendications 1 à 7, **caractérisé en ce que** l'alliage contient également un matériau renforcé par des fibres.

9. Procédé de fabrication de l'alliage selon l'une quelconque des revendications 1 à 8, **caractérisé en ce qu'**une ou plusieurs résines époxyde sont mélangées avec le mélange à des températures inférieures à la température de début de durcissement du composant agent de durcissement a).

10. Utilisation de l'alliage selon l'une quelconque des revendications 1 à 8 pour la fabrication de résines époxyde durcies.

11. Utilisation selon la revendication 10, **caractérisée en ce que** les résines époxyde durcies sont des corps moulés.

12. Utilisation selon la revendication 11, **caractérisée en ce que** les corps moulés contiennent un matériau renforçant.

13. Résine époxyde durcie pouvant être obtenue par durcissement de l'alliage selon l'une quelconque des revendications 1 à 8.

14. Résine époxyde durcie selon la revendication 13, **caractérisée en ce que** la résine époxyde durcie est un corps moulé.

15. Corps moulé selon la revendication 14, **caractérisé en ce qu'**il est renforcé par des fibres.

16. Corps moulé selon la revendication 15, pouvant être obtenu par durcissement d'un moule qui a été revêtu avec un matériau renforcé par des fibres et introduction de l'alliage selon l'une quelconque des revendications 1 à 7 par une technologie de perfusion dans le moule.

17. Corps moulé selon l'une quelconque des revendications 15 à 16, **caractérisé en ce que** le corps moulé constitue des composants renforcés pour pales de rotor d'éoliennes.
